# EUROPÄISCHE PATENTSCHRIFT

(11) **EP 2 349 185 B1**
(45) Veröffentlichungstag und Bekanntmachung des Hinweises auf die Patenterteilung: **24.01.2018**
(21) Anmeldenummer: 09780241.7
(22) Anmeldetag: 07.07.2009
(51) Int. Cl.: A61K 8/04, A61K 8/73, A61K 8/25, A61K 8/34, A61K 8/11, A61Q 15/00

(54) **WASSERFREIE DEODORANT-ZUSAMMENSETZUNGEN MIT VERBESSERTER LEISTUNG, DIE ALS SPRAY APPLIZIERT WERDEN**
MORE EFFECTIVE ANHYDROUS DEODORANT COMPOSITIONS APPLIED AS AN AEROSOL
COMPOSITIONS DE DÉODORANT ANHYDRES À EFFICACITÉ RENFORCÉE, APPLIQUÉES SOUS FORME D'AÉROSOL

(30) Priorität: 25.07.2008 DE 102008035014
(43) Veröffentlichungstag der Anmeldung: 03.08.2011
(73) Patentinhaber: Henkel AG & Co. KGaA, 40589 Düsseldorf (DE)
(72) Erfinder: BANOWSKI, Bernhard, 40597 Düsseldorf (DE); BREUER, Imme, 40474 Düsseldorf (DE)
(86) Internationale Anmeldenummer: PCT/EP2009/058583
(87) Internationale Veröffentlichungsnummer: WO 2010/009977

(56) Entgegenhaltungen:
- EP-A1- 1 576 946
- EP-A1- 1 738 803
- EP-A2- 1 428 520
- WO-A2-2006/128622
- US-A- 5 135 747
- US-A1- 2004 001 891
- US-A1- 2004 047 822

## Beschreibung

Gegenstand der vorliegenden Erfindung sind Wirkstoffkapseln mit deodorierender Wirkung, die ausgewählte aromatische Alkohole enthalten.

Ein weiterer Gegenstand der vorliegenden Erfindung sind wasserfreie Deodorant-Zusammensetzungen mit verbesserter Deodorantleistung, die als Spray appliziert werden, die verkapselte aromatische Alkohole enthalten.

Deodorant-Zusammensetzungen sind ein wichtiger Bestandteil der täglichen persönlichen Hygiene. Sie sollen dafür sorgen, dass der im Laufe des Tages durch diverse Aktivitäten (körperliche Bewegung, Arbeit, Sport), aber auch durch psychische Belastung gebildete Schweiß nicht zu unangenehmem Körpergeruch führt.

Genau so vielfältig, wie die Bestandteile des Schweißes und die Ursachen für die Körpergeruchsentwicklung sind, sind auch die deodorierenden Wirkstoffe der handelsüblichen Deodorantien. Als kosmetische deodorierende Wirkstoffe einsetzbar sind Geruchsabsorber, Duftstoffe, desodorierend wirkende Ionenaustauscher, keimhemmende Mittel, präbiotisch wirksame Komponenten sowie Enzyminhibitoren.

Körpergeruch ist, vereinfacht dargestellt, auf die bakterielle Zersetzung der organischen Bestandteile des Schweißes zurückzuführen. Für die bakterielle Zersetzung wiederum sind einige der für die natürliche Mikroflora der menschlichen Haut typischen Bakterien verantwortlich, insbesondere Gram-positive anaereobe Kokken, z. B. Staphylococcen, wie Staphylococcus hominis, und Corynebakterien.

Da der Körpergeruch durch bakterielle Tätigkeit entsteht, kann er besonders wirksam durch die Anwendung kosmetischer Mittel (Seifen, Cremes, Puder, Stifte, Roller, Gele oder Sprays) verhindert werden, die antimikrobiell wirksame Stoffe und Parfümölkompositionen enthalten. Als antimikrobiell wirksame Stoffe werden zur Herstellung von Desodorantien unter anderen Triclosan und Chlorhexidine verwendet. Weiter werden als Wirkstoffe für Desodorantien ätherische Öle wie Nelkonöl (Eugenol), Pfefferminzöl (Menthol) und Thymianöl (Thymol) verwendet, wobei der ausgeprägte Eigengeruch dieser Verbindungen jedoch dosislimitierend wirkt.

Als chlororganische Verbindung ist Triclosan ökologisch nicht unumstritten. Ätherische Öle haben ein relativ hohes allergenes Potenzial, was, zusätzlich zu ihrem Eigengeruch, ihren Einsatz einschränkt. Aromatische Alkohole, wie beispielsweise 2-Methyl-5-phenyl-pentan-1-ol, 2-Phenylethan-1-ol oder 3-Phenylpropan-1-ol, sind im Stand der Technik bereits als antimikrobielle und deodorierende Wirkstoffe bekannt. Diese Verbindungen sind hoch wirksam, zugleich sehr gut hautverträglich, und weisen nur einen schwachen Eigengeruch auf. Bei der üblichen Körperpflege, bei der das Deodorant direkt im Anschluss an die Körperreinigung auf die Haut aufgetragen wird, besteht allerdings direkt nach der Applikation noch gar kein Bedarf an einem antimikrobiellen Wirkstoff. Erst im Lauf des Tages erwächst bei fortgeschrittener Schweißbildung auf der feuchtwarmen Haut ein entsprechender Bedarf an antimikrobiellen, deodorierenden Wirkstoffen. Doch zu diesem Zeitpunkt hat ein Teil des applizierten Wirkstoffs bereits seine Aktivität eingebüßt, beispielsweise durch eine bereits erfolgte Einwirkung auf die eigentlich erwünschte, nicht geruchsbildende Hautmikroflora. So befindet sich im wirklichen Bedarfsfall nur noch eine nicht ausreichende Menge an Deodorant-Wirkstoff auf der Haut, und trotz eigentlich guter Körperpflege entsteht Körpergeruch.

WO 2006/128622 A2 offenbart wasserfreie Deodorant-Sprays, die verkapselte Deodorantwirkstoffe enthalten. Die Kapseln werden aus einem Polymer, vorzugsweise einem Polysaccharid, einem Emulgator und dem zu verkapselnden Parfüm hergestellt. Es gibt keinen Hinweis auf Silica (INCI: Silica) und Mannitol als Kapselbestandteile. US 5135747 offenbart wasserfreie Deodorant-Zusammensetzungen, darunter Sprays, die verkapselte Deodorantwirkstoffe enthalten. Die Kapseln werden aus einem Polymer, vorzugsweise einem Polysaccharid, und dem zu verkapselnden Parfüm hergestellt, ohne Hinweis auf Silica und Mannitol als Kapselbestandteile. US 2004/001891 A1 offenbart verkapselte Deodorantwirkstoffe, deren Kapselmaterial ein voll hydrolysiertes Polymer, modifizierte Maisstärke und hydrophobes Silica umfasst. Das hydrophobe Silica ist Silica, das mit einem Silicon modifiziert wurde, um eine Wasser abweisende Wirkung zu erzielen.

Aufgabe der vorliegenden Anmeldung war es daher, eine deodorierende kosmetische Zusammensetzung mit zeitlich verlängerter Wirkung der darin enthaltenen Deodorantwirkstoffe bzw. mit einer Wirkung "on demand", das heißt, erst im Bedarfsfall (= bei einsetzender Transpiration) bereitzustellen.

Überraschend wurde gefunden, dass die gestellte Aufgabe gelöst wird durch wasserfreie Zusammensetzungen, die einen deodorierend wirkenden aromatischen Alkohol enthalten, der mit ausgewählten wasserlöslichen Kapselmaterialien verkapselt ist.

Unter Feuchtigkeitseinfluss öffnet sich eine gewisse Zeit nach der Applikation das wasserlösliche Kapselmaterial, und der zuvor verkapselte deodorierende Wirkstoff wird freigesetzt. Die Freisetzung erfolgt also erst im Bedarfsfall, das heißt, bei beginnender Transpiration, und nicht schon direkt nach der Applikation auf die - zunächst - trockene Haut.

Alle Angaben über die Aggregatzustände der verwendeten Ausgangsstoffe (fest, flüssig...) in dieser Anmeldung beziehen sich auf Normalbedingungen. "Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Der Begriff "wasserfrei" wird erfindungsgemäß so verstanden, dass die Zusammensetzungen 0 bis maximal 3 Gew.-%, bevorzugt 0 bis maximal 2 Gew.-%, freies Wasser enthalten, bezogen auf die gesamte Zusammensetzung. Der Gehalt an Kristallwasser, Hydratationswasser oder ähnlich molekular gebundenem Wasser, der in den eingesetzten Bestandteilen, insbesondere in gegebenenfalls enthaltenen schweißhemmenden Wirkstoffen, enthalten sein kann, stellt im Sinne der vorliegenden Anmeldung kein freies Wasser dar.

Ein erster Gegenstand der vorliegenden Erfindung sind Wirkstoffkapseln mit deodorierender Wirkung, die mindestens einen deodorierend wirkenden aromatischen Alkohol der Struktur (AA-1) enthält, 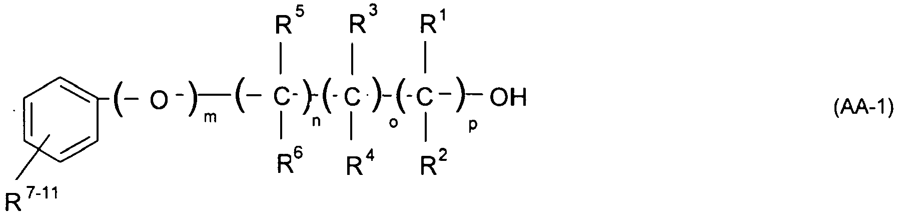 der verkapselt ist mit einer Mischung aus mindestens einem Polysaccharid, mindestens einer Polyhydroxyverbindung mit 4 bis 20 Kohlenstoffatomen, die unter Normalbedingungen fest ist, und mindestens einem teilchenförmigen Füllstoff, der ausgewählt ist aus Kieselsäuren, Silikaten, insbesondere Schichtsilikaten, sowie Mischungen hiervon,
wobei die Struktur (AA-1) folgendermaßen gekennzeichnet ist:
R¹ - R⁶ = unabhängig voneinander ein Wasserstoffatom, eine Alkylgruppe mit 1 bis 10 Kohlenstoffatomen, die linear oder verzweigt sein kann und die substituiert sein kann mit OH-Gruppen oder Alkoxy-Gruppen mit 1 bis 5 Kohlenstoffatomen, oder eine Alkenylgruppe mit 2 bis 10 Kohlenstoffatomen, die linear oder verzweigt sein kann und die substituiert sein kann mit OH-Gruppen oder Alkoxy-Gruppen mit 1 bis 5 Kohlenstoffatomen,
R⁷ - R¹¹ = unabhängig voneinander ein Wasserstoffatom, ein Halogenatom, insbesondere ein Chloratom, oder eine Alkylgruppe mit 1 bis 10 Kohlenstoffatomen, die linear oder verzweigt sein kann und die substituiert sein kann mit OH-Gruppen oder Alkoxy-Gruppen mit 1 bis 5 Kohlenstoffatomen, insbesondere mit einer Methoxygruppe,
m = 0 oder 1 ist,
n, o, p = unabhängig voneinander ganze Zahlen von 0 bis 10 sein können, wobei mindestens einer der Werte n, o, p ≠ 0 ist, dadurch gekennzeichnet, dass der deodorierend wirkende aromatische Alkohol der Struktur (AA-1) ausgewählt ist aus 2-Benzylheptan-1-ol, und dass der mindestens eine deodorierend wirkende aromatische Alkohol AA-1 verkapselt ist mit einer Mischung aus Natriumstärkeoctenylsuccinat, Mannitol und Kieselsäure (INCI: Silica).

| | m | n | o | p | R¹ | R² | R³ | R¹ | R⁵ | R⁶ | R⁷⁻¹¹ | |
|---|---|---|---|---|---|---|---|---|---|---|---|---|
| 2-Benzylheptan-1-ol | 0 | 1 | 1 | 1 | H | H | H | n-C₅H₁₁ | H | H | H | |

Besonders bevorzugte Alkohole der Struktur AA-1 sind solche, bei denen m = 0 ist.

Weitere besonders bevorzugte Alkohole der Struktur AA-1 sind solche, bei denen mindestens einer der Reste R¹, R², R³, R⁴, R⁵ oder R⁶ ≠ H ist.

Weitere besonders bevorzugte Alkohole der Struktur AA-1 sind solche, bei denen m = 0 und mindestens einer der Reste R¹, R², R³, R⁴, R⁵ oder R⁶ ≠ H ist.

Erfindungsgemäß besonders bevorzugte Wirkstoffkapseln sind dadurch gekennzeichnet, dass der deodorierend wirkende aromatische Alkohol der Struktur (AA-1) ausgewählt ist aus 2-Benzylheptan-1 -ol.

Im Hinblick auf Vorteile bei der Herstellung der Kapseln (möglichst problemlose Sprühtrocknung, mechanische Stabilität der Kapseln), dem Einsatz in den erfindungsgemäßen kosmetischen Zusammensetzungen (Lagerstabilität, Stabilität der Kapselwand gegenüber mechanischer und chemischer Belastung (z. B. pH-Stabilität), geringe Diffusionsverluste) und der Anwendung auf der Haut (geringe taktile Wahrnehmbarkeit bei der Applikation, Rückstandsverhalten des Kapselmaterials (z.B. geringe Klebrigkeit des Rückstands, möglichst geringer Transfer des Kapselmaterials im Kontakt mit der Kleidung, geringe Sichtbarkeit) hat sich Natriumstärkeoctenylsuccinat als besonders bevorzugtes Kapselmaterial erwiesen. Ein weiteres besonders bevorzugtes Kapselmaterial ist Natriumdextrinoctenylsuccinat.

Es hat sich als erfindungsgemäß günstig herausgestellt, dass das Kapselmaterial zusätzlich zu dem mindestens einen Polysaccharid mindestens eine Polyhydroxyverbindung mit 4 bis 20 Kohlenstoffatomen, die unter Normalbedingungen fest ist, enthält. Es wurde überraschend festgestellt, dass der Einsatz von mindestens einer Polyhydroxyverbindung mit 4 bis 20 Kohlenstoffatomen, die unter Normalbedingungen fest ist, im Gemisch mit dem verkapselnden Polysaccharid nicht nur die technischen Eigenschaften der Polysaccharid-Kapseln, beispielsweise im Hinblick auf die mechanische Stabilität, die Lagerstabilität oder das Verhalten bei der Sprühtrocknung, verbessert, sondern auch zu einer Verringerung der Klebrigkeit des Rückstands, zu einer Verringerung des Transfers des Kapselmaterials im Kontakt mit der Kleidung nach der Applikation auf die Haut, und/oder zu einer Steigerung der deodorierenden Wirkung des aromatischen Alkohols AA-1 führt.

Erfindungsgemäß bevorzugte kosmetische Zusammensetzungen sind dadurch gekennzeichnet, dass die mindestens eine Polyhydroxyverbindung a)i)2) ausgewählt ist aus Mannitol jeweils sowohl in der D-Form als auch in der L-Form sowie als Racemat. Mischungen der vorgenannten Substanzen können erfindungsgemäß bevorzugt sein.

Bevorzugt beträgt das Gewichtsverhältnis der Gesamtmenge an Verkapselungspolysaccharid(en) zur Gesamtmenge an Polyhydroxyverbindung(en) a)i)2) von 1:1 bis 10:1, besonders bevorzugt von 2:1 bis 6:1, besonders bevorzugt von 3:1 bis 5:1.

Weiterhin hat sich als erfindungsgemäß günstig herausgestellt, dass das Kapselmaterial zusätzlich zu dem mindestens einen Polysaccharid mindestens einen teilchenförmigen Füllstoff, ausgewählt aus Kieselsäuren, Silikaten, insbesondere Schichtsilikaten, sowie aus Mischungen hiervon, enthält. Überraschend wurde festgestellt, dass die Beimischung von mindestens einem der vorstehend genannten teilchenförmigen Füllstoffe die Anwendungseigenschaften der Wirkstoffkapseln hinsichtlich einer weiter verbesserten bzw. verlängerten Deodorantwirkung des verkapselten aromatischen Alkohols unterstützt.

Bevorzugte Kieselsäuren sind ausgewählt aus Aerogelkieselsäuren, pyrogenen Kieselsäuren, Gelkieselsäuren, Hydrogelkieselsäuren und Fällungskieselsäuren sowie Mischungen hiervon, wobei Fällungskieselsäuren erfindungsgemäß besonders bevorzugt sind. Gelkieselsäuren werden durch Umsetzung von Natriumsilikatlösungen mit starken wässrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die so genannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des so genannten Xerogels. Eine zweite, bevorzugt geeignete Gruppe von teilchenförmigen Füllstoffen sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Bevorzugt geeignet sind Fällungskieselsäuren mit einer BET-Oberfläche von 15 - 110 m²/g und einer Partikelgröße von 0,5 - 20 µm, wobei wenigstens 80 Gew.-% der Primärpartikel unter 5 µm liegen sollen. Bevorzugt geeignete Fällungskieselsäuren dieser Art weisen außerdem gerundete Ecken und Kanten auf und sind unter der Handelsbezeichnung Sident® 12 DS (DEGUSSA) erhältlich. Andere Fällungskieselsäuren dieser Art sind Sident® 8 (DEGUSSA) und Sorbosil® AC 39 (Crosfield Chemicals). Diese Kieselsäuren zeichnen sich durch eine etwas höhere mittlere Teilchengröße von 8 - 14 µm bei einer spezifischen Oberfläche von 40 - 75 m²/g (nach BET) aus. Eine weitere besonders bevorzugte Kieselsäure ist das Handelsprodukt SB-705 der Firma Miyoshi Kasei, eine sphärische Kieselsäure mit der INCI-Bezeichnung Silica, die einen mittleren Teilchendurchmesser von 5 - 6 µm und eine Oberfläche von etwa 600 m²/g aufweist.

Weitere bevorzugte pyrogene Kieselsäuren sind als Handelsprodukt Aerosil von Evonik Degussa erhältlich: Aerosil 130, Aerosil 200, Aerosil 255, Aerosil 300 oder Aerosil 380. Weitere bevorzugte Kieselsäuren sind ebenfalls als Handelsprodukte erhältlich: CAB-O-SIL Fumed Silica (Cabot), CAB-O-SIL EH-5 (Cabot), CAB-O-SIL HS-5 (Cabot), CAB-O-SIL LM-130 (Cabot), CAB-O-SIL MS-55 (Cabot), CAB-O-SIL M-5 (Cabot), Cosmedia Silc (Cognis), Neosil PC 50 S (Ineos Silicas), Sorbosil BFG 54 (Ineos Silicas), Sorbosil AC33 (Ineos Silicas), Sorbosil AC 35 (Ineos Silicas), Sorbosil AC 37 (Ineos Silicas), Sorbosil AC 39 (Ineos Silicas), Wacker HDK H 30 (Wacker-Chemie), Wacker HDK N 20 (Wacker-Chemie), Wacker HDK S 13 (Wacker-Chemie), Wacker HDK T 30 (Wacker-Chemie), Wacker HDK V 15 (Wacker-Chemie).
- Besonders bevorzugte Schichtsilicate sind ausgewählt aus Montmorillonit, Kaolinit, Illit, Beidellit, Nontronit, Saponit, Hectorit, Bentonit, Smectit und Talkum. Weitere bevorzugte Silikatfüllstoffe sind ausgewählt aus Magnesiumaluminiumsilikaten.

Bevorzugte Kapselmaterialien sind dadurch gekennzeichnet, dass das Gewichtsverhältnis der Gesamtmenge an Verkapselungspolysaccharid(en) zur Gesamtmenge an teilchenförmigen Füllstoffen, ausgewählt aus Kieselsäuren und Silikaten, von 10:1 bis 100:1, besonders bevorzugt von 20:1 bis 60:1, besonders bevorzugt von 30:1 bis 50:1, beträgt.

Weitere bevorzugte Kapselmaterialien sind dadurch gekennzeichnet, dass das Gewichtsverhältnis der Gesamtmenge an Verkapselungspolysaccharid(en) zur Gesamtmenge an Polyhydroxyverbindung(en) b)i)2) zur Gesamtmenge an teilchenförmigen Füllstoffen, ausgewählt aus Kieselsäuren und Silikaten, (1-10) : (0,5-2) : (0,05-0,5), bevorzugt (3-8) : (0,8-1,5) : (0,1-0,2), besonders bevorzugt (4-6): (1-1,3): (0,1-0,2), beträgt.

Bevorzugte Kapseln sind dadurch gekennzeichnet, dass das Gewichtsverhältnis der Gesamtmenge an Verkapselungspolysaccharid(en) zur Gesamtmenge an verkapseltem/n aromatischen Alkohol(en) der Struktur (AA-1) von 0,1 - 5, besonders bevorzugt 0,5 - 2, besonders bevorzugt 0,7 - 1,3, beträgt.

Weitere bevorzugte Kapseln sind dadurch gekennzeichnet, dass das Gewichtsverhältnis der Summe an Verkapselungspolysaccharid(en) und Polyhydroxyverbindung(en) a)i)2) zur Gesamtmenge an verkapseltem/n aromatischen Alkohol(en) der Struktur (AA-1) von 0,1 - 5, besonders bevorzugt 0,8 - 2, besonders bevorzugt 0,9 - 1,2, beträgt.

Weitere bevorzugte Kapseln sind dadurch gekennzeichnet, dass das Gewichtsverhältnis der Summe an Verkapselungspolysaccharid(en), Polyhydroxyverbindung(en) a)i)2) und teilchenförmigen Füllstoffen, ausgewählt aus Kieselsäuren und Silikaten, zur Gesamtmenge an verkapseltem/n aromatischen Alkohol(en) der Struktur (AA-1) von 0,3 - 3, besonders bevorzugt 0,6 - 2, besonders bevorzugt 0,9 - 1,5, beträgt.

Die erfindungsgemäßen Kapseln sind dadurch gekennzeichnet, dass der mindestens eine deodorierend wirkende aromatische Alkohol AA-1 verkapselt ist mit einer Mischung aus Natriumstärkeoctenylsuccinat, Mannitol und Kieselsäure (INCI: Silica).

Zur Erleichterung der großtechnischen Herstellung der erfindungsgemäßen Wirkstoffkapseln kann das Kapselmaterial in einer besonders bevorzugten Ausführungsform der Erfindung mindestens einen Emulgator enthalten. Als Emulgatoren sind nicht-ionische Emulgatoren, und hierbei insbesondere die hydrophilen nicht-ionischen Emulgatoren, d. h. solche mit einem HLB-Wert > 8, geeignet. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seite 913-916, aufgelistet sind. Für ethoxylierte Produkte wird der HLB-Wert erfindungsgemäß nach folgender Formel berechnet: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist. Der O/W-Emulgator wird in einer Menge von 0,1 - 10 Gew.-%, bevorzugt 0,1 - 5 Gew.-% und insbesondere 0,5 - 3 Gew.-% bezogen auf das Gesamtgewicht des Kapselmaterials, eingesetzt. Auch der Einsatz eines Gemisches von O/W-Emulgatoren kann vorteilhaft sein.

Erfindungsgemäß besonders bevorzugt ist es, eine Kombination von nichtionischen Emulgatoren einzusetzen. Zu den geeigneten nichtionischen Emulgatoren gehören beispielsweise:
(1) Anlagerungsprodukte von 2 bis 50 Mol Ethylenoxid und/oder 0 bis 20 Mol Propylenoxid an lineare Fettalkohole mit 8 bis 40 C-Atomen, an Fettsäuren mit 12 bis 40 C-Atomen oder an Alkylphenole mit 8 bis 15 C-Atomen in der Alkylgruppe;
(2) Phosphorsäuretriester von C₆ - C₂₂-Alkoholen sowie deren Ethylenoxidanlagerungsprodukte;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga.
(5) Anlagerungsprodukte von 15 bis 60 Mol Ethylenoxid an Ricinusöl und/oder gehärtetes Ricinusöl;
(6) C_{12/18}-Fettsäuremono- und -diester von Anlagerungsprodukten von 1 bis 50 Mol Ethylenoxid an Glycerin;
(7) Polyol- und insbesondere Polyglycerinester, wie z. B. Polyglycerinpolyricinoleat, Polyglycerinpoly-12-hydroxystearat oder Polyglycerindimerat; ebenfalls geeignet sind Gemische von Verbindungen aus mehreren dieser Substanzklassen;
(8) Polyalkylenglykole;
(9) Mischester aus Pentaerythrit, Fettsäuren, Citronensäure und Fettalkohol gemäß DE 1165574 und/oder Mischester von Fettsäuren mit 6 bis 22 Kohlenstoffatomen, Methylglucose und Polyolen, vorzugsweise Glycerin oder Polyglycerin.

In einer erfindungsgemäß bevorzugten Ausführungsform enthält das Verkapselungsmaterial mindestens einen nichtionischen Emulgator mit einem HLB-Wert von > 8 bis 18.

Besonders bevorzugte nicht-ionische Emulgatoren mit einem HLB-Wert > 8 sind Glycerinmono- und Difettsäureester, die Fettsäureester von Sorbitol und von Mono- und Disacchariden, insbesondere von Sucrose sowie deren alkoxylierten Derivaten, sowie die Phosphorsäuretriester von ethoxylierten C₆ - C₂₂ - Alkoholen.

Die Verkapselung kann nach bekannten Verfahren erfolgen. Hierzu kann beispielsweise eine homogene wässrige Dispersion aus den Bestandteilen des Kapselmaterials hergestellt werden, nämlich dem mindestens einen Polysaccharid, der mindestens einen Polyhydroxyverbindung mit 4 bis 20 Kohlenstoffatomen, die unter Normalbedingungen fest ist, dem mindestens einen teilchenförmigen Füllstoff, ausgewählt aus Kieselsäuren, Silikaten, insbesondere Schichtsilikaten, sowie Mischungen hiervon, und dem mindestens einen deodorierend wirkenden aromatischen Alkohol der Struktur (AA-1), wobei die Struktur (AA-1) durch folgende Parameter gekennzeichnet ist:
R1 - R6 = unabhängig voneinander ein Wasserstoffatom, eine Alkylgruppe mit 1 bis 10 Kohlenstoffatomen, die linear oder verzweigt sein kann und die substituiert sein kann mit OH-Gruppen oder Alkoxy-Gruppen mit 1 bis 5 Kohlenstoffatomen, oder eine Alkenylgruppe mit 2 bis 10 Kohlenstoffatomen, die linear oder verzweigt sein kann und die substituiert sein kann mit OH-Gruppen oder Alkoxy-Gruppen mit 1 bis 5 Kohlenstoffatomen,
R7 - R11 = unabhängig voneinander ein Wasserstoffatom, ein Halogenatom, insbesondere ein Chloratom, oder eine Alkylgruppe mit 1 bis 10 Kohlenstoffatomen, die linear oder verzweigt sein kann und die substituiert sein kann mit OH-Gruppen oder Alkoxy-Gruppen mit 1 bis 5 Kohlenstoffatomen, insbesondere mit einer Methoyxgruppe, m = 0 oder 1 ist, n, o, p = unabhängig voneinander ganze Zahlen von 0 bis 10 sein können, wobei mindestens einer der Werte n, o, p ≠ 0 ist,
   wobei optional mindestens ein Emulgator, optional bevorzugt mindestens ein nicht-ionischer Emulgator, optional besonders bevorzugt mindestens ein nicht-ionischer Emulgator mit einem HLB-Wert > 8 enthalten ist, und wobei alle Kapselbestandteile in den gewünschten Gewichtsverhältnissen zueinander eingewogen werden. Alle vorgenannten Kapselbestandteile werden in einer Menge Wasser dispergiert, die einerseits eine ausreichende Homogenisierung und gute Einspeisung in den Sprühtrockner gewährleistet und andererseits im Hinblick auf den Energieeinsatz bei der Sprühtrocknung nicht zu hoch gewählt ist. Zur Sprühtrocknung sind kommerziell erhältliche Sprühtrockenanlagen geeignet.

Bevorzugte Wirkstoffkapseln weisen eine Teilchengröße von maximal 100 µm, bevorzugt von maximal 90 µm, besonders bevorzugt maximal 75 µm, außerordentlich bevorzugt von maximal 60 µm, auf.

Weitere bevorzugte Wirkstoffkapseln weisen eine volumenmittlere Teilchengröße von 10 - 100 µm, bevorzugt 15 - 80 µm, besonders bevorzugt 20 - 60 µm, außerordentlich bevorzugt 25 - 50 µm, auf.

In einem anderen Herstellverfahren erfolgt die Wirkstoffverkapselung durch Koazervation.

Ein weiterer Gegenstand der vorliegenden Erfindung sind wasserfreie kosmetische Zusammensetzungen, enthaltend die vorstehend beschriebenen erfindungsgemäßen Wirkstoffkapseln und einen wasserfreien, unter Normalbedingungen flüssigen Träger, wobei die Zusammensetzungen als versprühbare Suspensionen konfektioniert und in einem Sprühspender verpackt sind.

Der mindestens eine erfindungsgemäß verkapselte, deodorierend wirkende aromatische Alkohol der Struktur (AA-1) ist, inclusive aller obligatorischen Kapselbestandteile a)i)1-3, in einer Gesamtmenge von 0,05 - 10 Gew.-%, bevorzugt 0,1 - 5 Gew.-%, besonders bevorzugt 0,2 - 3 Gew.-%, außerordentlich bevorzugt 0,3 - 1,5 Gew.-%, jeweils bezogen auf das Gesamtgewicht der treibmittelfreien erfindungsgemäßen Zusammensetzung, enthalten.

In einer besonders bevorzugten Ausführungsform enthalten die erfindungsgemäßen Zusammensetzungen - zur Unterstützung der Gesamtdeodorant-Leistung des Produktes - mindestens einen schweißhemmenden Wirkstoff, der auch als Antitranspirant-Wirkstoffe bezeichnet wird. Bevorzugte Antitranspirant-Wirkstoffe sind ausgewählt aus den wasserlöslichen adstringierenden anorganischen und organischen Salzen des Aluminiums, Zirkoniums und Zinks bzw. beliebigen Mischungen dieser Salze.

Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffs in 95 g Wasser bei 20 °C löslich sind.

Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.

Die Herstellung bevorzugter Antitranspirant-Wirkstoffe ist beispielsweise in US 3887692, US 3904741, US 4359456, GB 2048229 und GB 1347950 offenbart.

Weiterhin bevorzugt sind Aluminiumsesquichlorhydrat, Aluminiumdichlorhydrat, Aluminiumchlorhydrex-Propylenglykol (PG) oder Aluminiumchlorhydrex-Polyethylenglykol (PEG), Aluminium- oder Aluminiumzirkonium-Glycol-Komplexe, z. B. Aluminium- oder Aluminiumzirkonium-Propylenglycol-Komplexe, Aluminiumsesquichlorhydrex-PG oder Aluminiumsesquichlorhydrex-PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexen von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat, Zirconyloxyhalogeniden, insbesondere Zirconyloxychloriden, Zirconylhydroxyhalogeniden, insbesondere Zirconylhydroxychloriden (Zirkoniumchlorohydrat).

Erfindungsgemäß besonders bevorzugte Antitranspirant-Wirkstoffe sind ausgewählt aus so genannten "aktivierten" Aluminium- und Aluminium-Zirconiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart. Aktivierte Aluminium- und Aluminium-Zirconiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminium- und Aluminium-Zirconiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Aktivierte Aluminium- und Aluminium-Zirconiumsalze müssen nicht notwendigerweise als sprühgetrocknetes Pulver eingesetzt werden. Erfindungsgemäß ebenfalls bevorzugte schweißhemmende Wirkstoffe sind nicht-wässrige Lösungen oder Solubilisate eines aktivierten schweißhemmenden Aluminium- oder Aluminium-Zirconiumsalzes, beispielsweise gemäß US 6010688, die durch den Zusatz einer wirksamen Menge eines mehrwertigen Alkohols, der 3 bis 6 Kohlenstoffatome und 3 bis 6 Hydroxyl-Gruppen, bevorzugt Propylenglycol, Sorbit und Pentaerythrit, aufweist, gegen den Verlust der Aktivierung gegen den raschen Abbau des HPLC-Peak 4:Peak 3-Flächenverhältnisses des Salzes stabilisiert sind. Beispielsweise bevorzugt sind Zusammensetzungen, die in Gewichtsprozent (USP) enthalten: 18 - 45 Gew.-% eines aktivierten Aluminium- oder Aluminium-Zirconiumsalzes, 55 - 82 Gew.-% mindestens eines wasserfreien mehrwertigen Alkohols mit 3 bis 6 Kohlenstoffatomen und 3 bis 6 Hydroxyl-Gruppen, bevorzugt Propylenglycol, Butylenglycol, Diethylenglycol, Dipropylenglycol, Glycerin, Sorbit und Pentaerythrit, besonders bevorzugt Propylenglycol.

Besonders bevorzugt sind auch Komplexe aktivierter schweißhemmender Aluminium- oder Aluminium-Zirconiumsalze mit einem mehrwertigen Alkohol, die 20 - 50 Gew.-%, besonders bevorzugt 20 - 42 Gew.-%, aktiviertes schweißhemmendes Aluminium- oder Aluminium-Zirconiumsalz und 2 - 16 Gew.-% molekular gebundenes Wasser enthalten, wobei der Rest zu 100 Gew.-% mindestens ein mehrwertiger Alkohol mit 3 bis 6 Kohlenstoffatome und 3 bis 6 Hydroxyl-Gruppen ist. Propylenglycol, Propylenglycol/Sorbit-Mischungen und Propylenglycol/Pentaerythrit-Mischungen sind bevorzugte derartige Alkohole. Derartige erfindungsgemäß bevorzugte Komplexe eines aktivierten schweißhemmenden Aluminium- oder Aluminium-Zirconiumsalzes mit einem mehrwertigen Alkohol sind z. B. offenbart in US 5643558 und US 6245325.

Weitere bevorzugte schweißhemmende Wirkstoffe sind basische Calcium-Aluminiumsalze, wie sie beispielsweise in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind Aluminium-Zirconium-Komplexe, wie sie beispielsweise in US 4017599 offenbart sind, die mit Salzen von Aminosäuren, insbesondere mit Alkali- und Erdalkaliglycinaten, gepuffert sind.

Weitere bevorzugte schweißhemmende Wirkstoffe sind aktivierte Aluminium- oder Aluminium-Zirconiumsalze, wie sie beispielsweise in US 6245325 oder US 6042816 offenbart sind, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Calciumsalz in einer solchen Menge, um ein Ca:(Al+Zr)-Gewichtsverhältnis von 1:1 - 1:28 und bevorzugt 1:2 - 1:25 bereitzustellen. Besonders bevorzugte feste aktivierte schweißhemmende Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Calciumsalz, dass das Ca:(Al+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminium- oder Aluminium-Zirconiumsalze, wie sie beispielsweise in US 6902723 offenbart sind, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Strontiumsalz in einer solchen Menge, um ein Sr:(Al+Zr)-Gewichtsverhältnis von 1:1 - 1:28 und bevorzugt 1:2 - 1:25 bereitzustellen.

Besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(Al+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(Al+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(Al+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von AI:X 0,9:1 bis 2,1:1 beträgt, wie sie beispielsweise in US 6074632 offenbart sind. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Bevorzugte aktivierte Aluminium-Zirconiumsalze sind solche, die Mischungen oder Komplexe der vorstehend beschriebenen Aluminiumsalze mit Zirconiumsalzen der Formel ZrO(OH)_{2-pb}Y_{b} darstellen, worin Y Cl, Br, I, NO₃ oder SO₄ ist, b eine rationale Zahl von 0,8 bis 2 und p die Wertigkeit von Y ist, wie sie beispielsweise in US 6074632 offenbart sind. Die Zirconiumsalze haben in der Regel ebenfalls etwas Hydratationswasser assoziativ gebunden, typischerweise 1 bis 7 Mol Wasser pro Mol Salz. Vorzugsweise ist das Zirconiumsalz Zirconylhydroxychlorid mit der Formel ZrO(OH)_{2-b}Cl_{b}, worin b eine rationale Zahl von 0,8 bis 2, bevorzugt 1,0 bis 1,9 ist. Bevorzugte Aluminium-Zirconiumsalze haben ein Al:Zr-Molverhältnis von 2 bis 10 und ein Metall:(X+Y)-Verhältnis von 0,73 bis 2,1, bevorzugt 0,9 bis 1,5. Ein besonders bevorzugtes Salz ist Aluminium-Zirconiumchlorhydrat (d.h., X und Y sind Cl), das ein Al:Zr-Verhältnis von 2 bis 10 und ein molares Metall:Cl-Verhältnis von 0,9 bis 2,1 hat. Der Begriff Aluminium-Zirconiumchlorhydrat umfasst die Tri-, Tetra-, Penta- und Octachlorhydratformen.

Erfindungsgemäß bevorzugte Zirconiumsalze haben die allgemeine Formel ZrO(OH)₂₋ₐClₐ · x H₂O mit a = 1.5 - 1.87; x = 1 - 7, wobei a und x rationale Zahlen sind. Diese Zirconiumsalze sind beispielsweise in der belgischen Schrift BE 825146 offenbart.

Weitere bevorzugte schweißhemmende Wirkstoffe sind in US 6663854 und US 20040009133 offenbart.

Die schweißhemmenden Wirkstoffe können sowohl in solubilisierter als auch in ungelöster, suspendierter Form vorliegen.

Sofern die schweißhemmenden Wirkstoffe in einem mit Wasser nicht mischbaren Träger suspendiert vorliegen, ist es aus Gründen der Produktstabilität bevorzugt, dass die Wirkstoffpartikel eine zahlenmittlere Partikelgröße von 0,1 - 200 µm, bevorzugt 1 - 50 µm, besonders bevorzugt 3 - 20 µm und außerordentlich bevorzugt 5 - 10 µm, aufweisen.

Bevorzugte Aluminiumsalze und Aluminiumzirconiumsalze weisen ein molares Metall-zu-Chlorid-Verhältnis von 0,9 - 1,3, bevorzugt 0,9 - 1,1, besonders bevorzugt 0,9 - 1,0, auf.

Bevorzugte Aluminiumzirconiumchlorohydrate haben im allgemeinen die empirische Formel AlₙZr(OH)_{[3n+4-m(n+1)]}(Cl)_{[m(n+1)]} mit n = 2,0 - 10,0, bevorzugt 3,0 - 8,.0, m = 0,77 - 1,11 (entsprechend einem molaren Metall (Al+Zr)-zu-Chlorid-Verhältnis von 1,3 - 0,9), bevorzugt m = 0,91 - 1,11 (entsprechend M:CI = 1,1 - 0,9), und besonders bevorzugt m = 1,00 - 1,11 (entsprechend M:CI = 1,0 - 0,9), weiterhin sehr bevorzugt m = 1,02 - 1,11 (entsprechend M:CI = 0,98 - 0,9) sowie sehr bevorzugt m = 1,04 - 1,11 (entsprechend M:CI = 0,96 - 0,9).

Bei diesen Salzen ist im Allgemeinen etwas Hydratationswasser assoziativ gebunden, typischerweise 1 - 6 Mol Wasser pro Mol Salz, entsprechend 1 - 16 Gew.-%, bevorzugt 4 - 13 Gew.-% Hydratationswasser.

Üblicherweise sind die bevorzugten Aluminiumzirconiumchlorohydrate mit einer Aminosäure assoziiert, um die Polymerisation der Zirconiumspecies während der Herstellung zu verhindern. Bevorzugte stabilisierende Aminosäuren sind ausgewählt aus Glycin, Alanin, Leucin, Isoleucin, β-Alanin, Cystein, Valin, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt. Die Aminosäure ist in einer Menge von 1 - 3 Mol, bevorzugt 1,3-1,8 Mol, jeweils pro Mol Zirconium in dem Salz enthalten.

Bevorzugte schweißhemmende Salze sind Aluminium-Zirconiumtetrachlorohydrat (AI:Zr = 2-6; M:CI = 0.9-1.3), insbesondere Salze mit einem molaren Metall-zu-Chlorid-Verhältnis von 0,9 - 1,1, bevorzugt 0,9 - 1,0.

Weiterhin erfindungsgemäß bevorzugt sind Aluminiumzirconiumchlorohydrat-Glycin-Salze, die mit Betain ((CH₃)₃N⁺-CH₂-COO⁻) stabilisiert sind. Besonders bevorzugte entsprechende Verbindungen weisen ein molares Gesamt-(Betain + Glycin)/Zr-Verhältnis von (0,1 - 3,0) : 1, bevorzugt (0,7 - 1,5) : 1 und ein molares Verhältnis von Betain zu Glycin von mindestens 0,001 : 1 auf. Entsprechende Verbindungen sind beispielsweise offenbart in US 7105691.

In einer besonders bevorzugten erfindungsgemäßen Ausführungsform ist als besonders wirksames Antitranspirant-Salz ein so genanntes "aktiviertes" Salz enthalten, insbesondere eines mit einem hohen HPLC-Peak 5-Aluminium-Gehalt, insbesondere mit einer Peak 5-Fläche von mindestens 33 %, besonders bevorzugt mindestens 45 %, bezogen auf die gesamte Fläche unter den Peaks 2 - 5, gemessen mit HPLC einer 10 Gew.-%igen wässrigen Lösung des Wirkstoffs unter Bedingungen, bei denen die Aluminiumspecies in mindestens 4 aufeinander folgende Peaks aufgelöst werden (mit Peaks 2 - 5 bezeichnet). Bevorzugte Aluminiumzirconiumsalze mit einem hohen HPLC-Peak 5-Aluminium-Gehalt (auch als "E⁵AZCH" bezeichnet) sind beispielsweise offenbart in US 6436381 und US 6649152.

Weiterhin sind solche aktivierten "E⁵AZCH"-Salze bevorzugt, deren HPLC-Peak 4-zu-Peak 3-Flächenverhältnis von mindestens 0,4, bevorzugt mindestens 0,7, besonders bevorzugt mindestens 0,9, beträgt.

Weitere besonders bevorzugte Antitranspirant-Wirkstoffe sind solche Aluminiumzirconiumsalze mit einem hohen HPLC-Peak 5-Aluminium-Gehalt, die zusätzlich mit einem wasserlöslichen Strontiumsalz und/oder mit einem wasserlöslichen Calciumsalz stabilisiert sind. Entsprechende Salze sind beispielsweise in US 6923952 offenbart.

Weitere bevorzugte Antitranspirant-Wirkstoffe sind ausgewählt aus adstringierenden Titansalzen, wie sie beispielsweise in GB 2299506 A offenbart sind.

Die Antitranspirant-Wirkstoffe können als nicht-wässrige Lösungen oder als glycolische Solubilisate eingesetzt werden.

Besonders bevorzugte erfindungsgemäße Zusammensetzungen sind dadurch gekennzeichnet, dass der mindestens eine Antitranspirant-Wirkstoff in einer Menge von 5 - 40 Gew.-%, bevorzugt 10 - 35 Gew.-% und besonders bevorzugt 20 - 30 Gew.-%, enthalten ist, bezogen auf das Gesamtgewicht der kristallwasserfreien Aktivsubstanz (USP) in der treibmittelfreien Gesamtzusammensetzung.

In einer besonders bevorzugten Ausführungsform enthält die Zusammensetzung ein adstringierendes Aluminiumsalz, insbesondere Aluminiumchlorohydrat, das beispielsweise pulverförmig als Micro Dry® Ultrafine oder Microdry von Reheis, Microdry 323 von Summit als Chlorhydrol® sowie in aktivierter Form als Reach® 501 von Reheis vertrieben wird. Unter der Bezeichnung Reach® 301 wird ein Aluminiumsesquichlorohydrat von Reheis angeboten, das ebenfalls besonders bevorzugt ist. Besonders bevorzugt sind auch aktivierte Aluminiumchlorohydrate, die unter den Bezeichnungen Reach® 101 und Reach® 103 von Reheis oder Summit erhältlich sind. Auch die Verwendung von Aluminium-Zirkonium-Tetrachlorohydrex-Glycin-Komplexen, die beispielsweise von Reheis unter der Bezeichnung Rezal® 36 GP oder, in aktivierter Qualität, als Reach® 908, als Pulver im Handel sind, kann erfindungsgemäß besonders bevorzugt sein.

Die Konfektionierung der erfindungsgemäßen Zusammensetzungen, die als Spray appliziert werden, richtet sich vorzugsweise nach den Anforderungen der gewünschten Sprayapplikation. Die erfindungsgemäßen Zusammensetzungen liegen als Suspension vor, das heißt, die Wirkstoffkapseln und gegebenenfalls weitere unlösliche Bestandteile sind in einem flüssigen Träger suspendiert. Ein solches disperses System sollte vor der Anwendung geschüttelt werden. Bevorzugte erfindungsgemäße Zusammensetzungen können z. B. in Pump- oder Quetschspendern abgepackt sein, insbesondere in Mehrkammer-Pump- oder Quetschspendern. Derartige Spender verwenden Luft, insbesondere die Umgebungsluft, als Treibmittel bzw. fördern die erfindungsgemäße Zusammensetzung durch Pumpen.

In einer weiteren bevorzugten Ausführungsform der Erfindung wird die Zusammensetzung mittels eines komprimierten Treibmittels appliziert.

Alle Mengenangaben beziehen sich, sofern nicht anders angegeben, auf das Gewicht der treibmittelfreien Zusammensetzung.

Die Verpackung in einem Mehrkammerspender bietet besondere technische Vorteile. Da die Stabilität der den mindestens einen aromatischen Alkohol der Struktur (AA-1) enthaltenden Polysaccharidkapseln gemäß Anspruch 1 nicht nur durch Wasser selbst, sondern auch durch bestimmte, von Wasser verschiedene, Lösemittel bedroht sein kann, kann es vorteilhaft sein, die Kapseln in einem inerten, vorzugsweise flüssigen Träger, in einer ersten Kammer der Verpackung physikalisch getrennt von den übrigen Bestandteilen der erfindungsgemäßen Deodorantzusammensetzung, die in einer zweiten Kammer bevorratet sind, aufzubewahren, um die Lagerstabilität der gesamten Zusammensetzung zu gewährleisten. Beide Kammern sind so miteinander verbunden, dass beide Teilzusammensetzungen gleichzeitig aus der Verpackung ausgegeben werden können, beispielsweise aus getrennten Öffnungen oder aus einer einzigen Öffnung. So sind einerseits die Lagerstabilität des gesamten erfindungsgemäßen Mittels und andererseits eine hohe Aktivität der verkapselten Wirkstoffe auch nach längerer Lagerung gewährleistet.

Der Mehrkammerspender kann auch so eingesetzt werden, dass eine Kammer mit der erfindungsgemäßen Zusammensetzung befüllt ist, während eine andere Kammer das komprimierte Treibmittel enthält. Ein derartiger Mehrkammerspender ist beispielsweise eine so genannte Bag-in-Can-Verpackung.

Erfindungsgemäß bevorzugte Deodorant- oder Antitranspirant-Zusammensetzungen enthalten 30 - 95 Gew.-%, bevorzugt 40 - 93 Gew.-%, besonders bevorzugt 50 - 90 Gew.-%, außerordentlich bevorzugt 55 - 85 Gew.-%, jeweils bezogen auf die gesamte treibmittelfreie Zusammensetzung, an mindestens einem unter Normalbedingungen flüssigen kosmetischen Öl. Erfindungsgemäß bevorzugte kosmetische Öle sind ausgewählt aus Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Besonders bevorzugt sind flüchtige Siliconöle, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls besonders bevorzugt sind flüchtige lineare Siliconöle, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/oder L₄, bevorzugt solche Mischungen, wie sie z. B. in den Handelsprodukten DC 2-1184, Dow Corning® 200 (0,65 cSt) und Dow Corning® 200 (1,5 cSt) von Dow Corning enthalten sind.

Flüchtige Siliconöle sind hervorragend geeignete Trägeröle für erfindungsgemäß bevorzugte Deodorant- oder Antitranspirant-Zusammensetzungen, da sie ihnen ein angenehmes Hautgefühl und eine geringe Kleideranschmutzung verleihen. Erfindungsgemäß besonders bevorzugte Deodorant- oder Antitranspirant-Zusammensetzungen sind daher durch einen Gehalt an mindestens einem flüchtigen Siliconöl von 30 - 95 Gew.-%, bevorzugt 40 - 93 Gew.-%, besonders bevorzugt 50 - 90 Gew.-%, außerordentlich bevorzugt 55 - 85 Gew.-%, jeweils bezogen auf die gesamte treibmittelfreie Zusammensetzung, gekennzeichnet.

Neben oder anstelle des mindestens einen flüchtigen Siliconöls kann auch mindestens ein flüchtiges Nichtsiliconöl enthalten sein. Bevorzugte flüchtige Nichtsiliconöle sind ausgewählt aus C₈-C₁₆-Isoparaffinen, insbesondere aus Isodecan, Isododecan, Isotetradecan und Isohexadecan sowie Mischungen hiervon. Auch dieses mindestens eine flüchtige Nichtsiliconöl ist bevorzugt in einer Gesamtmenge von 30 - 95 Gew.-%, bevorzugt 40 - 93 Gew.-%, besonders bevorzugt 50 - 90 Gew.-%, außerordentlich bevorzugt 55 - 85 Gew.-%, jeweils bezogen auf die gesamte treibmittelfreie Zusammensetzung, enthalten.

Aufgrund des trockeneren Hautgefühls und der schnelleren Wirkstofffreisetzung sind Siliconöle als flüchtiges Trägeröl gegenüber Isoparaffinen besonders bevorzugt.

Neben den vorgenannten, üblicherweise als "flüchtigen" Siliconölen bezeichneten Substanzen sowie neben den vorgenannten flüchtigen Nichtsiliconölen können erfindungsgemäß besonders bevorzugte Deodorant- oder Antitranspirant-Zusammensetzungen weiterhin mindestens ein nichtflüchtiges kosmetisches Öl, ausgewählt aus nichtflüchtigen Siliconölen und nichtflüchtigen Nichtsiliconölen, enthalten. Das mindestens eine nichtflüchtige Öl gleicht den negativen Effekt des flüchtigen Öls auf das Rückstandsverhalten erfindungsgemäß bevorzugter Deodorant- oder Antitranspirant-Zusammensetzungen aus. Durch die relativ schnelle Verdunstung der flüchtigen Öle können feste, unlösliche Bestandteile, insbesondere die Antitranspirantwirkstoffe, auf der Haut als unschöner Rückstand sichtbar werden. Diese Rückstände können erfolgreich mit einem nichtflüchtigen Öl maskiert werden. Außerdem können mit einem Gemisch aus nichtflüchtigem und flüchtigem Öl Parameter wie Hautgefühl, Sichtbarkeit des Rückstands und Stabilität der Suspension feingesteuert und besser an die Bedürfnisse der Verbraucher angepasst werden.

Selbstverständlich ist es ebenfalls möglich, wasserfreie Deodorant- oder Antitranspirant-Zusammensetzungen mit einem geringen Anteil an flüchtigen Ölen oder sogar ohne flüchtige Öle zu formulieren.

Bevorzugte nichtflüchtige Siliconöle sind ausgewählt aus höhermolekularen linearen Dimethylpolysiloxanen, im Handel erhältlich z. B. unter der Bezeichnung Dow Corning® 190, Dow Corning® 200 Fluid mit kinematischen Viskositäten (25°C) im Bereich von 5 - 100 cSt, bevorzugt 5 - 50 cSt oder auch 5 - 10 cSt, und Baysilon® 350 M (mit einer kinematischen Viskosität (25°C) von etwa 350 cSt. Erfindungsgemäß ebenfalls bevorzugte nichtflüchtige Siliconöle sind ausgewählt aus Siliconen der Formel (Sil-1), wobei x ausgewählt ist aus ganzen Zahlen von 1 - 20, bevorzugt 1 - 3.

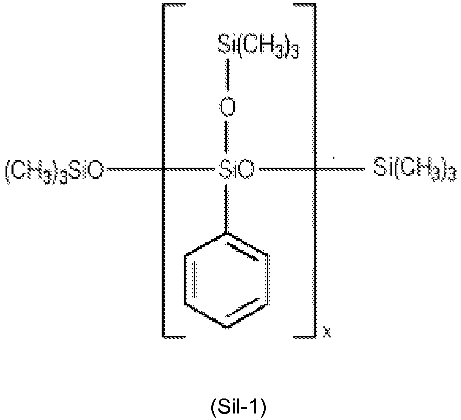

Ein bevorzugtes Siliconöl der Formel (Sil-1) ist erhältlich unter der INCI-Bezeichnung Phenyl Trimethicone.

Natürliche und synthetische Kohlenwasserstoffe, wie beispielsweise Paraffinöle, C₁₈-C₃₀-Isoparaffine, insbesondere Isoeicosan, Polyisobutene oder Polydecene, die beispielsweise unter der Bezeichnung Emery® 3004, 3006, 3010 oder unter der Bezeichnung Ethylflo® von Albemarle oder Nexbase® 2004G von Nestle erhältlich sind, sowie 1,3-Di-(2-ethylhexyl)-cyclohexan (erhältlich z. B. unter dem Handelsnamen Cetiol®S von Cognis) gehören ebenfalls zu den erfindungsgemäß bevorzugten nichtflüchtigen Nichtsiliconölen.

Weitere erfindungsgemäß bevorzugte nichtflüchtige Nichtsiliconöle sind ausgewählt aus den Benzoesäureestern von linearen oder verzweigten C₈₋₂₂-Alkanolen. Besonders bevorzugt sind Benzoesäure-C12-C15-alkylester, z. B. erhältlich als Handelsprodukt Finsolv® TN, Benzoesäureisostearylester, z. B. erhältlich als Handelsprodukt Finsolv® SB, Ethylhexylbenzoat, z. B. erhältlich als Handelsprodukt Finsolv® EB, und Benzoesäureoctyldocecylester, z. B. erhältlich als Handelsprodukt Finsolv® BOD. Derartige Benzoesäureesteröle sind besonders gut zur Maskierung von Antitranspirant-Wirkstoff-Rückständen geeignet, da ihr Brechungsindex den besonders wirksamen Aluminium-Zirkonium-Mischsalzen sehr nahe kommt. Außerdem weisen diese Öle ein angenehmes Hautgefühl auf. Außerdem ermöglichen die Benzoesäureester-Öle aufgrund ihrer Polarität eine gute Wirkstofffreisetzung von wasserlöslichen Deodorant- oder Antitranspirant-Wirkstoffen aus den erfindungsgemäßen Zusammensetzungen heraus.

Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol® G 16, Guerbitol® T 16), Octyldodecanol (Eutanol® G, Guerbitol® 20), 2-Ethylhexylalkohol und die Handelsprodukte Guerbitol® 18, Isofol® 12, Isofol® 16, Isofol® 24, Isofol® 36, Isocarb® 12, Isocarb® 16 oder Isocarb® 24.

Weitere bevorzugte nichtflüchtige Nichtsiliconöle sind ausgewählt aus Mischungen aus Guerbetalkoholen und Guerbetalkoholestern, z.B. dem Handelsprodukt Cetiol® PGL (Hexyldecanol und Hexyldecyllaurat).

Weitere erfindungsgemäß bevorzugte nichtflüchtige Nichtsiliconöle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol® 318, Myritol® 331 (Cognis) oder Miglyol® 812 (Hüls) mit unverzweigten Fettsäureresten sowie Glyceryltriisostearin und die Handelsprodukte Estol® GTEH 3609 (Uniqema) oder Myritol® GTEH (Cognis) mit verzweigten Fettsäureresten.

Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den Estern der linearen oder verzweigten gesättigten oder nichtflüchtige Nichtsiliconöle ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Hexyldecylstearat (Eutanol® G 16 S), Hexyldecyllaurat, Isodecylneopentanoat, Isononylisononanoat, 2-Ethylhexylpalmitat (Cegesoft® C 24) und 2-Ethylhexylstearat (Cetiol® 868). Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und -dipalmitat.

Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether (Witconol® APM).

Weitere erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether (Ucon Fluid® AP), PPG-9-Butylether (Breox® B25), PPG-10-Butandiol (Macol® 57), PPG-15-Stearylether (Arlamol® E) und Glycereth-7-diisononanoat.

Weitere erfindungsgemäß besonders bevorzugte nichtflüchtige Nichtsiliconöle sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂-C₁₅-Alkyllactat, und von in 2-Position verzweigten C_{12/13}-Alkanolen sind unter dem Warenzeichen Cosmacol® von der Firma Nordmann, Rassmann GmbH & Co, Hamburg, zu beziehen, insbesondere die Handelsprodukte Cosmacol® ESI, Cosmacol® EMI und Cosmacol® ETI.

Weitere erfindungsgemäß besonders bevorzugte nichtflüchtige Nichtsiliconöle sind ausgewählt aus den symmetrischen, unsymmetrischen oder cyclischen Estern der Kohlensäure mit Fettalkoholen, z. B. Glycerincarbonat, Dicaprylylcarbonat (Cetiol® CC) oder die Ester gemäß der Lehre der DE 19756454 A1.

Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Es kann erfindungsgemäß bevorzugt sein, Mischungen der vorgenannten Öle einzusetzen.

Bevorzugte erfindungsgemäße Zusammensetzungen sind dadurch gekennzeichnet, dass das unter Normalbedingungen flüssige Trägeröl ausgewählt ist flüchtigen Siliconölen, nichtflüchtigen Siliconölen, flüchtigen Kohlenwasserstoffölen, verzweigten gesättigten oder ungesättigten Fettalkoholen mit 6 - 30 Kohlenstoffatomen, Triglyceriden von linearen oder verzweigten, gesättigten oder ungesättigten, gegebenenfalls hydroxylierten C₈₋₃₀-Fettsäuren, Dicarbonsäureestern von linearen oder verzweigten C₂-C₁₀-Alkanolen, Estern von verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können, Anlagerungsprodukten von 1 bis 5 Propylenoxid-Einheiten an ein- oder mehrwertige C₈₋₂₂-Alkanole, Anlagerungsprodukten von mindestens 6 Ethylenoxid und/oder Propylenoxid-Einheiten an ein- oder mehrwertige C₃₋₂₂-Alkanole, C₈-C₂₂-Fettalkoholestern einwertiger oder mehrwertiger C₂-C₇-Hydroxycarbonsäuren, symmetrischen, unsymmetrischen oder cyclischen Estern der Kohlensäure mit Fettalkoholen, den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen, sowie Mischungen der vorgenannten Substanzen.

Es kann erfindungsgemäß außerordentlich bevorzugt sein, Mischungen der vorgenannten Öle einzusetzen, um eine optimale Feinabstimmung der Produkteigenschaften, insbesondere des Rückstandsverhaltens, des Hautgefühls oder der Wirkstofffreisetzung, zu erzielen.

Weitere erfindungsgemäß bevorzugte Zusammensetzungen sind dadurch gekennzeichnet, dass sie mindestens eine unter Normalbedingungen feste Fettkomponente mit einem Schmelzpunkt von mehr als 80 - 150 °C enthalten, die partikelförmig suspendiert vorliegt.

Bevorzugte Fettkomponenten mit einem Schmelzpunkt von > 80 - 150 °C sind ausgewählt aus Wachsen. Generell sind Wachse von fester bis brüchig harter Konsistenz, grob bis feinkristallin, durchscheinend bis opak, jedoch nicht glasartig, und schmelzen oberhalb von 50 °C ohne Zersetzung. Sie sind schon wenig oberhalb des Schmelzpunktes niedrigviskos und zeigen eine stark temperaturabhängige Konsistenz und Löslichkeit.

Erfindungsgemäß bevorzugt sind beispielsweise natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Polyalkylenwachse, insbesondere Polyethylenwachse, und Polyethylenglycolwachse, C₂₀-C₄₀-Dialkylester von Dimersäuren, C₃₀-₅₀-Alkylbienenwachs sowie Alkyl- und Alkylarylester von Dimerfettsäuren.

Eine besonders bevorzugte Wachskomponente ist ausgewählt aus mindestens einem Ester aus einem gesättigten, einwertigen C₁₆-C₆₀-Alkohol und einer gesättigten C₈-C₃₆-Monocarbonsäure. Erfindungsgemäß zählen hierzu auch Lactide, die cyclischen Doppelester von α-Hydroxycarbonsäuren der entsprechenden Kettenlänge. Ester aus Fettsäuren und langkettigen Alkoholen haben sich für die erfindungsgemäß bevorzugten Zusammensetzungen als besonders vorteilhaft erwiesen, weil sie ausgezeichnete sensorische Eigenschaften und - den Stiften - eine hohe Stabilität verleihen. Die Ester setzen sich aus gesättigten, verzweigten oder unverzweigten Monocarbonsäuren und gesättigten, verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten, verzweigten oder unverzweigten Alkoholen sind erfindungsgemäß einsetzbar, sofern die Wachskomponente einen Schmelzpunkt > 80°C hat. Besonders bevorzugt ist, die Wachskomponenten zu wählen aus der Gruppe der Ester aus gesättigten, verzweigten oder unverzweigten Alkancarbonsäuren einer Kettenlänge von 12 bis 24 C-Atomen und den gesättigten, verzweigten oder unverzweigten Alkoholen einer Kettenlänge von 16 bis 50 C-Atomen, die einen Schmelzpunkt > 80°C haben.

Insbesondere können als Wachskomponente C₁₆₋₃₆-Alkylstearate und C₁₈₋₃₈-Alkylhydroxystearoyl-stearate, C₂₀₋₄₀-Alkylerucate sowie Cetearylbehenat bevorzugt sein. Das Wachs oder die Wachskomponenten weisen einen Schmelzpunkt > 80°C, bevorzugt > 85°C, auf.

Eine besonders bevorzugte Ausführungsform der Erfindung enthält als Wachskomponente ein C₂₀-C₄₀-Alkylstearat. Dieser Ester ist unter den Namen Kesterwachs® K82H oder Kesterwachs® K80H bekannt und wird von Koster Keunen Inc. vertrieben. Es handelt sich um die synthetische Nachahmung der Monoesterfraktion des Bienenwachses und zeichnet sich durch seine Härte, seine Ölgelierfähigkeit und seine breite Kompatibilität mit Lipidkomponenten aus. Kesterwachs bietet den Vorteil, dass es auch bei geringen Konzentrationen eine exzellente Ölgelierfähigkeit aufweist und so die Stift- oder Soft Solid-Masse nicht zu schwer macht und einen samtigen Abrieb ermöglicht. Eine weitere besonders bevorzugte Ausführungsform der Erfindung enthält als Wachskomponente Cetearylbehenat, d. h. Mischungen aus Cetylbehenat und Stearylbehenat. Dieser Ester ist unter dem Namen Kesterwachs® K62 bekannt und wird von Koster Keunen Inc. vertrieben.

Weitere bevorzugte Wachskomponenten mit einem Schmelzpunkt > 80°C sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 50 °C aufweisen, beispielsweise bevorzugt C₁₈ - C₃₆ Acid Triglyceride (Syncrowax® HGL-C). Erfindungsgemäß ist als Wachskomponente hydriertes Rizinusöl, erhältlich z. B. als Handelsprodukt Cutina® HR, besonders bevorzugt.

Weitere besonders bevorzugte Wachskomponenten mit einem Schmelzpunkt > 80°C sind Polyethylenwachspartikel, insbesondere solche, die unter der Handelsbezeichnung Microthene® erhältlich sind.

Weitere bevorzugte erfindungsgemäße Zusammensetzungen sind dadurch gekennzeichnet, dass die Wachskomponente/n als Bestandteil des erfindungsgemäßen Trägers in einer Gesamtmenge von 0,1 - 5 Gew.-%, bevorzugt 0,2 - 1 Gew.-%, besonders bevorzugt 0,3 - 0,5 Gew.-%, jeweils bezogen auf die treibmittelfreie Gesamtzusammensetzung, enthalten ist.

Weitere bevorzugte erfindungsgemäße Zusammensetzungen sind dadurch gekennzeichnet, dass sie, beispielsweise zur Verbesserung der Konsistenz und der sensorischen Eigenschaften, zusätzlich zu dem Kieselsäure- oder Silikat-Füllstoff, der Bestandteil der erfindungsgemäßen Wirkstoffkapseln ist, noch mehr bzw. mindestens einen weiteren festen, wasserunlöslichen teilchenförmigen Füllstoff enthalten. In einer außerordentlich bevorzugten Ausführungsform ist dieser Füllstoff ausgewählt aus gegebenenfalls modifizierten Stärken (z. B. aus Mais, Reis, Kartoffeln) und Stärkederivaten, die gewünschtenfalls vorverkleistert sind, Siliciumdioxid, Kieselsäuren, z. B. Aerosil®-Typen, sphärischen Polyalkylsesquisiloxan-Partikeln (insbesondere Aerosil® R972 und Aerosil® 200V von Degussa), Kieselgelen, Talkum, Kaolin, Magnesiumaluminiumsilikaten, Bornitrid, Lactoglobulinderivaten, z. B. Natrium-C₈₋₁₆-Isoalkylsuccinyllactoglobulinsulfonat, von Brooks Industries erhältlich als Handelsprodukt Biopol® OE, Glaspulvern, Polymerpulvern, insbesondere aus Polyolefinen, Polycarbonaten, Polyurethanen, Polyamiden, z. B. Nylon, Polyestern, Polystyrolen, Polyacrylaten, (Meth)acrylat- oder (Meth)acrylat-Vinyliden-Copolymeren, die vernetzt sein können, oder Siliconen, sowie Mischungen dieser Substanzen.

Polymerpulver auf Basis eines Polymethacrylat-Copolymers sind z. B. als Handelsprodukt Polytrap® 6603 (Dow Corning) erhältlich. Andere Polymerpulver, z. B. auf Basis von Polyamiden, sind unter der Bezeichnung Orgasol® 1002 (Polyamid-6) und Orgasol® 2002 (Polyamid-12) von Elf Atochem erhältlich. Weitere Polymerpulver, die sich als erfindungsgemäß bevorzugte Füllstoffe eignen, sind z. B. Polymethacrylate (Micropearl® M von SEPPIC oder Plastic Powder A von NIKKOL), Styrol-Divinylbenzol-Copolymeren (Plastic Powder FP von NIKKOL), Polyethylen- und Polypropylen-Pulver (ACCUREL® EP 400 von AKZO) oder auch Siliconpolymere (Silicone Powder X2-1605 von Dow Corning).

Bevorzugte erfindungsgemäße Zusammensetzungen sind dadurch gekennzeichnet, dass sie mindestens einen festen, wasserunlöslichen teilchenförmigen Füllstoff in einer Gesamtmenge von 0,1 - 5 Gew.-%, bevorzugt 0,2 - 1 Gew.-%, besonders bevorzugt 0,3 - 0,5 Gew.-%, außerordentlich bevorzugt 0,6 - 0,9 Gew.-%, jeweils bezogen auf die treibmittelfreie Gesamtzusammensetzung, enthalten.

Weitere erfindungsgemäß bevorzugte Zusammensetzungen mindestens einen Öl-in-Wasser-Emulgator enthalten. Bevorzugte Öl-in-Wasser-Emulgatoren weisen einen HLB-Wert von mehr als 7 auf. Weitere bevorzugte Öl-in-Wasser-Emulgatoren sind nichtionisch. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seiten 913 - 916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist.

Bei der Auswahl erfindungsgemäß geeigneter nichtionischer Öl-in-Wasser-Emulgatoren ist es besonders bevorzugt, ein Gemisch von nichtionischen Öl-in-Wasser-Emulgatoren einzusetzen, um die Eigenschaften der erfindungsgemäßen Zusammensetzungen, wie Wirkstofffreisetzung oder Abwaschbarkeit, optimal einstellen zu können. Die einzelnen Emulgatorkomponenten liefern dabei einen Anteil zum Gesamt-HLB-Wert oder mittleren HLB-Wert des Öl-in-Wasser-Emulgatorgemisches gemäß ihrem Mengenanteil an der Gesamtmenge der Öl-in-Wasser-Emulgatoren. Die erfindungsgemäßen Zusammensetzungen, insbesondere die Deodorant- oder Antitranspirant-Stifte, können aber in einer anderen bevorzugten Ausführungsform auch nur einen einzigen Öl-in-Wasser-Emulgator, bevorzugt mit einem HLB-Wert im Bereich von 11 - 17, besonders bevorzugt 12 - 15 und außerordentlich bevorzugt 13 - 14, enthalten.

Bevorzugte erfindungsgemäße kosmetische Zusammensetzungen sind dadurch gekennzeichnet, dass die nichtionischen Öl-in-Wasser-Emulgatoren ausgewählt sind aus ethoxylierten C₈-C₂₄-Alkanolen mit durchschnittlich 10-100 Mol Ethylenoxid pro Mol, ethoxylierten C₈-C₂₄-Carbonsäuren mit durchschnittlich 10-100 Mol Ethylenoxid pro Mol, mit durchschnittlich 20-100 Mol Ethylenoxid pro Mol ethoxylierten Sorbitanmonoestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit n = 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, sofern sie einen HLB-Wert von mehr als 7 aufweisen, sowie Mischungen der vorgenannten Substanzen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet.

Die ethoxylierten C₈-C₂₄-Carbonsäuren haben die Formel R¹O(CH₂CH₂O)ₙH, wobei R¹O steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-40-monostearat, PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen, insbesondere Ceteth-10, Ceteth-12, Ceteth-20, Ceteth-30, Steareth-10, Steareth-12, Steareth-20, Steareth-30, Ceteareth-10, Ceteareth-12, Ceteareth-20, Ceteareth-30, Laureth-12 und Beheneth-20. Bevorzugte mit durchschnittlich 20 - 100 Mol Ethylenoxid pro Mol ethoxylierte Sorbitanmonoester von linearen gesättigten und ungesättigten C₁₂ - C₃₀-Carbonsäuren, die hydroxyliert sein können, sind ausgewählt aus Polysorbate-20, Polysorbate-40, Polysorbate-60 und Polysorbate-80.

Weiterhin werden vorzugsweise C₈ - C₂₂-Alkylmono- und -oligoglycoside eingesetzt. C₈ -C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare® erhältlich sind, enthalten eine glucosidisch gebundene C₈-C₁₆-Alkylgruppe an einem Oligoglucosidrest, dessen mittlerer Oligomerisationsgrad bei 1 - 2, insbesondere 1,2 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.

Weitere erfindungsgemäß bevorzugte Öl-in-Wasser-Emulgatoren sind ausgewählt aus Organosiloxan-Oxyalkylen-Copolymeren. Bevorzugte Organosiloxan-Oxyalkylen-Copolymer-O/W-Emulgatoren sind ausgewählt aus Verbindungen der allgemeinen Strukturformeln (I), (II), (III), (IV) und (V), 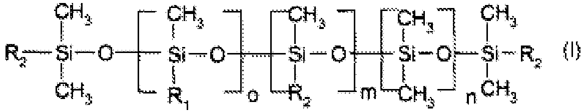 Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare® erhältlich sind, enthalten eine glucosidisch gebundene C₈-C₁₆-Alkylgruppe an einem Oligoglucosidrest, dessen mittlerer Oligomerisationsgrad bei 1 - 2, insbesondere 1,2 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat,Decaglycerintristearat, Decaglycerintrioleat und Decaglycerintrihydroxystearat.

Weitere erfindungsgemäß bevorzugte Öl-in-Wasser-Emulgatoren sind ausgewählt aus Organosiloxan-Oxyalkylen-Copolymeren. Bevorzugte Organosiloxan-Oxyalkylen-Copolymer-O/W-Emulgatoren sind ausgewählt aus Verbindungen der allgemeinen Strukturformeln (I), (II), (III) und (V), 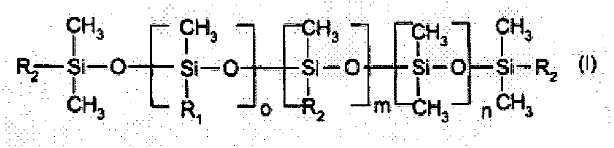 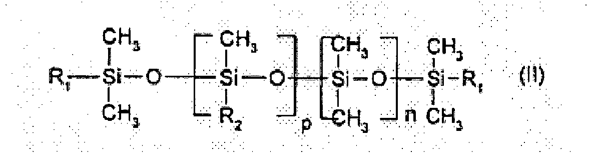 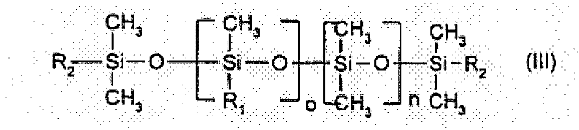 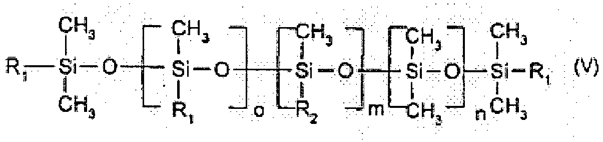 wobei
die Reste R¹ unabhängig voneinander eine lineare oder verzweigte C₁-C₃₀-Alkylgruppe oder eine ggf. substituierte Phenylgruppe darstellen,
die Reste R² unabhängig voneinander die Gruppen -C_{c}H_{2c}-O-(C₂H₄O-)ₐ(C₃H₆O-)_{b}R⁵ oder -C_{c}H_{2c}-O-(C₂H₄O-)ₐR⁵ darstellen,
die Reste R³ und R⁴ unabhängig voneinander eine lineare oder verzweigte C₁-C₁₆-Alkylgruppe und bevorzugt Methylgruppen darstellen,
die Reste R⁵ unabhängig voneinander ein Wasserstoffatom oder eine lineare oder verzweigte C₁-C₁₆-Alkylgruppe und bevorzugt ein Wasserstoffatom oder eine Methylgruppe darstellen,
m eine Zahl von 0 - 20 darstellt,
n eine Zahl von 0 - 500 darstellt,
o eine Zahl von 0 - 20 darstellt,
p eine Zahl von 1 - 50 darstellt,
a eine Zahl von 0 - 50 darstellt,
b eine Zahl von 0 - 50 darstellt,
a + b mindestens 1 sind,
c eine Zahl von 1 - 4, besonders bevorzugt 3, darstellt,
x eine Zahl von 1 - 100 darstellt.
n = 0
p = 1.

Ein derartiges Organosiloxan-Oxyalkylen-Copolymer ist beispielsweise unter der Handelsbezeichnung Silwet L-77 erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit
R¹ = Methyl,
R² = -C_{c}H_{2c}-O-(C₂H₄O-)ₐ(C₃H₆O-)_{b}R⁵
mit
a = 22
b = 24
c = 3
R⁵ = Methyl,
n = 10 - 500,
p = 10 - 50.

Ein derartiges Organosiloxan-Oxyalkylen-Copolymer ist beispielsweise in einer Mischung mit Cyclomethicone unter der Handelsbezeichnung Mirasil DMCO (INCI: Cyclomethicone, PEG/PPG-22/24 Dimethicone) erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit
R¹ = Methyl,
R² = -C_{c}H_{2c}-O-(C₂H₄O-)ₐ(C₃H₆O-)_{b}R⁵
mit
a = 17
b = 18
c = 3
R⁵ = Methyl,
n = 10 - 500,
p = 10 - 50.

Ein derartiges Organosiloxan-Oxyalkylen-Copolymer ist beispielsweise unter der Handelsbezeichnung Dow Corning Q2-5220 (INCI: PEG/PPG-17/18 Dimethicone) erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit
R¹ = Methyl,
R² = -C_{c}H_{2c}-O-(C₂H₄O-)ₐ(C₃H₆O-)_{b}R⁵
mit
a = 20
b=6
c=3
R⁵ = Methyl,
n = 10 - 500,
p = 5 - 50.

Ein derartiges Organosiloxan-Oxyalkylen-Copolymer ist beispielsweise unter der Handelsbezeichnung Abil B 88184 (INCI: PEG/PPG-20/6 Dimethicone) erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit
R¹ = Methyl,
R² = -C_{c}H_{2c}-O-(C₂H₄O-)ₐ(C₃H₆O-)_{b}R⁵
mit
a= 14
b=4
c=3
R⁵ = Methyl,
n = 10 - 500,
p = 5 - 50.

Ein derartiges Organosiloxan-Oxyalkylen-Copolymer ist beispielsweise unter der Handelsbezeichnung Abil B 8851 (INCI: PEG/PPG-14/4 Dimethicone) erhältlich.

Besonders bevorzugte erfindungsgemäße Zusammensetzungen sind dadurch gekennzeichnet, dass mindestens ein Öl-in-Wasser-Emulgator, bevorzugt mindestens ein nichtionischer Öl-in-Wasser-Emulgator, in einer Gesamtmenge von 0,5 - 10 Gew.-%, bevorzugt 1 - 9 Gew.-%, besonders bevorzugt 1,5 - 8,5 Gew.-%, außerordentlich bevorzugt 2 - 8 Gew.-%, weiterhin auch 2,5, 3, 3,5, 4,, 4,5, 5, 5,5, 6, 6,5, 7, 7,5, und 8 Gew.-%, bezogen auf die treibmittelfreie Gesamtzusammensetzung, enthalten ist.

Weitere bevorzugte erfindungsgemäße Zusammensetzungen sind dadurch gekennzeichnet, dass sie als Träger wasserfreies Ethanol und/oder mindestens einen wasserfreien mehrwertigen Alkohol mit 3 bis 9 Kohlenstoffatomen, der unter Normalbedingungen flüssig ist, enthalten. Dazu zählen 1,2-Propylenglycol, 2-Methyl-1,3-propandiol, Glycerin, 1,2-Butylenglycol, 1,3-Butylenglycol und 1,4-Butylenglycol, Pentylenglycole wie 1,2-Pentandiol und 1,5-Pentandiol, Hexandiole wie 1,2-Hexandiol und 1,6-Hexandiol, Hexantriole wie 1,2,6-Hexantriol, 2-Ethyl-2-hydroxymethyl-1,3-propandiol, Diethylenglycol, Triethylenglycol, Dipropylenglycol, Tripropylenglycol, Diglycerin und Triglycerin. Besonders bevorzugt sind wasserfreies Ethanol und/oder 1,2-Propylenglycol. Außerordentlich bevorzugt ist wasserfreies Ethanol, das 70 - 98 Gew.-%, bevorzugt 75 - 95 Gew.-%, der gesamten treibmittelfreien Zusammensetzung ausmacht.

Als Verdickungsmittel enthalten bevorzugte erfindungsgemäße Ethanol- oder Glycol-basierte Suspensionen mindestens eine Substanz, ausgewählt aus der Gruppe, umfassend Celluloseether, vor allem Hydroxyalkylcellulosen, insbesondere Hydroxypropylcellulose, Hydroxypropylmethylcellulose, Hydroxyethylcellulose, Carboxymethylcellulose, Cetylhydroxyethylcellulose, Hydroxybutylmethylcellulose, Methylhydroxyethylcellulose, weiterhin Xanthan-Gum, Sclerotium Gum, Succinoglucane, Polygalactomannane, insbesondere Guar-Gums und Johannisbrotkernmehl (Locust Bean Gum), insbesondere Guar-Gum und Locust Bean Gum selbst und die nichtionischen Hydroxyalkylguarderivate und Johannisbrotkernmehl-Derivate, wie Hydroxypropylguar, Carboxymethylhydroxypropylguar, Hydroxypropylmethylguar, Hydroxyethylguar und Carboxymethylguar, weiterhin Pectine, Agar, Carragheen (Carrageenan), Traganth, Gummi arabicum, Karayagummi, Taragummi, Gellan, Gelatine, Casein, Propylenglycolalginat, Alginsäuren und deren Salze, insbesondere Natriumalginat, Kaliumalginat und Calciumalginat. Erfindungsgemäß besonders bevorzugt ist Hydroxypropylcellulose. Dieses Verdickungsmittel ist bevorzugt in einer Gesamtmenge von 0,08 - 1 Gew.-%, bevorzugt 0,1 - 0,5 Gew.-%, besonders bevorzugt 0,2 - 0,4 Gew.-%, außerordentlich bevorzugt 0,3 Gew.-%, jeweils bezogen auf das Gesamtgewicht der treibmittelfreien Zusammensetzung, enthalten.

In einer weiteren bevorzugten Ausführungsform enthalten die erfindungsgemäßen Zusammensetzungen mindestens einen Komplexbildner. Als Komplexbildner besonders bevorzugt ist wasserfreie Ethylendiamintetraessigsäure (EDTA) und ihre Natriumsalze, insbesondere das Tetranatriumsalz und das Trinatriumsalz, wasserfreie 1,3-Propylendiamintetraessigsäure (PDTA) und ihre Natriumsalze, insbesondere das Tetranatriumsalz und das Trinatriumsalz, wasserfreie Hydroxyethylethylendiamintetraessigsäure (HEDTA) und ihre Natriumsalze, insbesondere das Tetranatriumsalz und das Trinatriumsalz, wasserfreie Diethylentriaminpentaessigsäure (DTPA) und ihre Natriumsalze, insbesondere das Pentanatriumsalz, das Tetranatriumsalz und das Trinatriumsalz, Nitrilotriessigsäure (NTA) und ihre Natriumsalze, β-Alanindiessigsäure und ihre Salze, das Ethanoldiglycindinatriumsalz, das Diethanolglycinnatriumsalz und Phosphonsäuren und deren Salze. Die mindestens eine komplexbildende Substanz ist bevorzugt in einer Gesamtmenge von 0,05 - 2 Gew.-%, besonders bevorzugt 0,1 - 1, außerordentlich bevorzugt 0,2 - 0,5 Gew.-%, jeweils bezogen auf das Gesamtgewicht der treibmittelfreien erfindungsgemäßen Zusammensetzung, enthalten.

In einer bevorzugten erfindungsgemäßen Ausführungsform sind die erfindungsgemäßen Wirkstoffkapseln in mindestens einem unter Normalbedingungen flüssigen Öl suspendiert. Zur besseren Anwendbarkeit wird dieser Suspension noch mindestens ein lipophiles Verdickungsmittel als Suspendierhilfe zugesetzt. Weitere bevorzugte erfindungsgemäße Zusammensetzungen sind daher dadurch gekennzeichnet, dass sie mindestens ein lipophiles Verdickungsmittel enthalten. Erfindungsgemäß bevorzugte lipophile Verdickungsmittel sind ausgewählt aus hydrophobierten Tonmineralien, pyrogenen Kieselsäuren, Ethylene/Propylene/Styrene Copolymeren, Butylene/ Ethylene/Styrene Copolymeren, Dextrinestern und Siliconelastomeren. Hierunter sind hydrophobierte Tonmineralien besonders bevorzugt.

Bevorzugte hydrophobierte Tonmineralien sind ausgewählt aus hydrophobierten Montmorilloniten, hydrophobierten Hectoriten und hydrophobierten Bentoniten, besonders bevorzugt aus Disteardimonium Hectorite, Stearalkonium Hectorite, Quaternium-18 Hectorite und Quaternium-18 Bentonite. Die handelsüblichen Verdickungsmittel stellen diese hydrophobierten Tonmineralien in Form eines Gels in einer Ölkomponente, bevorzugt in Cyclomethicone und/oder einer Nichtsilicon-Ölkomponente, wie z. B. Propylencarbonat, bereit. Die Gelbildung erfolgt durch den Zusatz geringer Mengen an Aktivatoren, wie insbesondere Ethanol oder Propylencarbonat, aber auch Wasser. Derartige Gele sind beispielsweise unter der Handelsbezeichnung Bentone® oder Thixogel erhältlich. Bevorzugte erfindungsgemäße Zusammensetzungen enthalten mindestens einen Aktivator in einer Gesamtmenge von 0,1 - 3 Gew.-%, bevorzugt 0,3 - 1,6 Gew.-%, jeweils bezogen auf das Gesamtgewicht der treibmittelfreien erfindungsgemäßen Zusammensetzung. Weitere bevorzugte erfindungsgemäße Zusammensetzungen enthalten mindestens einen Aktivator, ausgewählt aus Ethanol, Propylencarbonat und Wasser sowie Mischungen hiervon, in einer Gesamtmenge von 0,1 - 3 Gew.-%, bevorzugt 0,3 - 1,6 Gew.-%, jeweils bezogen auf das Gesamtgewicht der treibmittelfreien erfindungsgemäßen Zusammensetzung. Erfindungsgemäß bevorzugte Zusammensetzungen sind dadurch gekennzeichnet, dass sie mindestens ein hydrophobiertes Tonmineral in einer Gesamtmenge von 0,5 - 10 Gew.-%, bevorzugt 1 - 7 Gew.-%, besonders bevorzugt 2 - 6 Gew.-%, außerordentlich bevorzugt 3 - 5 Gew.-%, jeweils bezogen auf das Gesamtgewicht der treibmittelfreien erfindungsgemäßen Zusammensetzung, enthalten.

Weitere erfindungsgemäß bevorzugte lipophile Verdickungsmittel sind ausgewählt aus pyrogenen Kieselsäuren, z. B. den Handelsprodukten der Aerosil®-Serie von Evonik Degussa. Besonders bevorzugt sind hydrophobierte pyrogene Kieselsäuren, besonders bevorzugt Silica Silylate und Silica Dimethyl Silylate.

Erfindungsgemäß bevorzugte Zusammensetzungen sind dadurch gekennzeichnet, dass sie mindestens eine pyrogene Kieselsäure, bevorzugt mindestens eine hydrophobierte pyrogene Kieselsäure, in einer Gesamtmenge von 0,5 - 10 Gew.-%, bevorzugt 0,8 - 5 Gew.-%, besonders bevorzugt 1 - 4 Gew.-%, außerordentlich bevorzugt 1,5 - 2 Gew.-%, jeweils bezogen auf das Gesamtgewicht der treibmittelfreien erfindungsgemäßen Zusammensetzung, enthalten.

Weitere erfindungsgemäß bevorzugte Zusammensetzungen sind dadurch gekennzeichnet, dass sie mindestens eine hydrophobierte pyrogene Kieselsäure und mindestens eine hydrophile Kieselsäure enthalten.

Weitere erfindungsgemäß bevorzugte lipophile Verdickungsmittel sind ausgewählt aus Ethylene/Propylene/Styrene Copolymeren und Butylene/Ethylene/Styrene Copolymeren. Besonders bevorzugt werden die Copolymere als vorverdicktes öl-basiertes Gel eingesetzt. Derartige Gele sind beispielsweise unter der Handelsbezeichnung Versagel® (ex Penreco) erhältlich. Bevorzugt sind Gele mit Mineralöl, hydriertem Polyisobuten, Isoparaffinen, wie Isohexadecan oder Isododecan, sowie mit Esterölen, insbesondere mit Isopropylpalmitat oder Isopropylmyristat.

Erfindungsgemäß bevorzugte Zusammensetzungen sind dadurch gekennzeichnet, dass sie mindestens ein Ethylene/Propylene/Styrene Copolymer und/oder Butylene/Ethylene/Styrene Copolymer in einer Gesamtmenge von 0,05 - 3 Gew.-%, bevorzugt 0,1 - 2 Gew.-%, besonders bevorzugt 0,2 - 1,0 Gew.-%, außerordentlich bevorzugt 0,3 - 0,5 Gew.-%, jeweils bezogen auf das Gesamtgewicht der treibmittelfreien erfindungsgemäßen Zusammensetzung, enthalten.

Weitere erfindungsgemäß bevorzugte lipophile Verdickungsmittel sind ausgewählt aus Siliconelastomeren. Eine weitere bevorzugte Ausführungsform der Erfindung ist dadurch gekennzeichnet, dass mindestens ein Siliconelastomer, das erhältlich ist durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist, enthalten ist.

Erfindungsgemäß besonders bevorzugte Organopolysiloxane mit mindestens 2 C₂ - C₁₀ - AlkenylGruppen mit terminaler Doppelbindung im Molekül sind ausgewählt aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen. Erfindungsgemäß besonders bevorzugte vernetzende Organopolysiloxane mit mindestens zwei Silicon-gebundenen Wasserstoffatomen sind ausgewählt aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-siloxan-Copolymeren. Erfindungsgemäß besonders bevorzugte Siliconelastomere, die als Rohstoff bereits in einem bei Raumtemperatur unter Normalbedingungen flüssigen Silicon vorgequollen vorliegen und ein Silicon-basiertes Gel darstellen, sind kommerziell erhältlich, beispielsweise unter den Handelsnamen SFE 168, ein Cyclomethicone (and) Dimethicone/Vinyl Dimethicone Crosspolymer von GE Silicones, Vinyl Dimethicone Crosspolymere, enthalten in KSG-15 (Cyclomethicone (and) Dimethicone/Vinyl Dimethicone Crosspolymer, Siliconelastomergehalt 4 - 10 Gew.-%), KSG-16 (Dimethicone (and) Dimethicone/Vinyl Dimethicone Crosspolymer, Siliconelastomergehalt 20 - 30 Gew.-%), KSG-17 (Cyclomethicone (and) Dimethicone/Vinyl Dimethicone Crosspolymer), KSG-18 (Phenyl Trimethicone (and) Dimethicone/Phenyl Vinyl Dimethicone Crosspolymer, Siliconelastomergehalt 10 - 20 Gew.-%); and KSG-20, erhältlich von Shin Etsu Silicones of America (Akron, Ohio), und von Grant Industries Inc. (Elmwood Park, NJ) die Produkte aus der Gransil®-Serie, insbesondere Gransil SR-CYC (Cyclomethicone and Stearyl-Vinyl/hydromethylsiloxane Copolymer), Gransil® RPS Gel (INCI-Bezeichnung: Cyclopentasiloxane and Polysilicone-11), Gransil® GCM-4 (INCI-Bezeichnung: Cyclotetrasiloxane and Polysilicone-11), Gransil®GCM-5 (INCI-Bezeichnung: Cyclopentasiloxane and Polysilicone-11), Gransil® RPS (INCI-Bezeichnung: Cyclopentasiloxane and Polysilicone-11), GI-CD 10 (INCI-Bezeichnung: Cyclopentasiloxane (and) Stearoxymethicone/Dimethicone Copolymer (and) Dimethicone), Gransil® IDS (INCI-Bezeichnung: Isododecane (and) Cyclotetrasiloxane (and) Polysilicone-11), Gransil®PC-12 (INCI-Bezeichnung: Isododecane (and) Polysilicone-11), Gransil®IDS-5 (INCI-Bezeichnung: Isododecane (and) Cyclopentasiloxane (and) Polysilicone-11), Gransil®APK-1 (INCI-Bezeichnung: Dimethicone and Cyclopentasiloxane and Polysilicone-11 and Nylon-12 and Methyl Methacrylate/Acrylonitrile Copolymer and PEG-10 Dimethicone and Polysorbate-40 and Isohexadecane and Ammonium Polyacryloyldimethyl Taurate), Gransil®DMCM-5 (INCI-Bezeichnung: Dimethicone and Cyclopentasiloxane and Polysilicone-11), Gransil®DMG-6 mit Dimethicone (6 cSt) (INCI-Bezeichnung: Dimethicone and Polysilicone-11), Gransil®DMG-20 mit Dimethicone (20 cSt) (INCI-Bezeichnung: Dimethicone and Polysilicone-11), Gransil® AM-8 Gel (INCI-Bezeichnung: Caprylyl Methicone and Cyclopentasiloxane and Polysilicone-11), Gransil® DM 5 mit Dimethicone (5 cSt) (INCI-Bezeichnung: Dimethicone and Polysilicone-11), Gransil® DMID (INCI-Bezeichnung: Dimethicone and Isododecane and Polysilicone-11), Gransil® PM (INCI-Bezeichnung: Phenyl Trimethicone and Polysilicone-11), Gransil® ININ (INCI-Bezeichnung: Isononyl Isononanoate (and) Polysilicone-11).

Ebenfalls mit Vorzug in den erfindungsgemäßen Zusammensetzungen einsetzbar sind Siliconelastomere, die als Rohstoff bereits in einem bei Raumtemperatur unter Normalbedingungen flüssigen Silicon, gemischt mit einem Nicht-Silicon-haltigen Öl, vorgequollen vorliegen und ein Silicon-/Nicht-Silicon-basiertes Gel darstellen. Derartige Siliconelastomer-Zusammensetzungen sind ebenfalls kommerziell erhältlich, beispielsweise unter den Handelsnamen Gransil® MLB (INCI-Bezeichnung: Cyclopentasiloxane and Polysilicone-11 and Beeswax), Gransil® PS (INCI-Bezeichnung: Cyclotetrasiloxane and Polysilicone-11 and Petrolatum), Gransil® PS-5 (INCI-Bezeichnung: Cyclopentasiloxane and Polysilicone-11 and Petrolatum), Gransil® DMG-20 P mit Dimethicone (20 cSt) und Petrolatum (INCI-Bezeichnung: Dimethicone and Polysilicone-11 and Petrolatum), Gransil® RJO (INCI-Bezeichnung: Cyclopentasiloxane and Polysilicone-11 and Jojoba Oil), Gransil® LANO (INCI-Bezeichnung: Cyclopentasiloxane and Polysilicone-11 and Lanolin), Gransil® OHS-5 (INCI-Bezeichnung: Cyclopentasiloxane and Polysilicone-11 and Octyl Hydroxystearate) und Gransil® DML (INCI-Bezeichnung: Dimethicone (and) Neopentyl Glycol Diheptanoate (and) Polysilicone-11).

Eine weitere bevorzugte Ausführungsform der Erfindung ist dadurch gekennzeichnet, dass das Siliconelastomer durch die Vernetzung eines Organopolysiloxans, das mindestens 2 C₂ - C₁₀-Alkenylgruppen mit terminaler Doppelbindung in jedem Molekül enthält, mit mindestens einem alpha, omega-Dien erhältlich ist. Erfindungsgemäß besonders bevorzugte vernetzende alpha, omega-Diene weisen die Formel CH₂=CH(CH₂)ₓCH=CH₂ mit x = 1 - 20 auf. Besonders bevorzugte alpha, omega-Diene sind ausgewählt aus 1,4-Pentadien, 1,5-Hexadien, 1,6-Heptadien, 1,7-Octadien, 1,8-Nonadien, 1,11-Dodecadien, 1,13-Tetradecadien und 1,19- Eicosadien. Erfindungsgemäß bevorzugte Zusammensetzungen sind dadurch gekennzeichnet, dass sie mindestens ein Siliconelastomer in einer Gesamtmenge von 0,05 - 3 Gew.-%, bevorzugt 0,1 - 2 Gew.-%, besonders bevorzugt 0,15 - 0,5 Gew.-%, außerordentlich bevorzugt 0,2 - 0,3 Gew.-%, jeweils bezogen auf das Gesamtgewicht der treibmittelfreien erfindungsgemäßen Zusammensetzung, enthalten.

Weitere bevorzugte erfindungsgemäße Zusammensetzungen sind dadurch gekennzeichnet, dass sie mindestens ein Silicongummi enthalten. Überraschend wurde festgestellt, dass durch den Zusatz eines Silicongummis die deodorierende Wirkung der erfindungsgemäßen Zusammensetzungen weiter verlängert werden kann. Ohne an diese Theorie gebunden sein zu wollen, wird vermutet, dass das Silicongummi auf der Haut einen Film bildet, durch den die erfindungsgemäßen Wirkstoffkapseln besser auf der Haut haften.

Ein erfindungsgemäß besonders bevorzugtes Silicongummi ist ausgewählt aus Siliconpolymeren mit der INCI-Bezeichnung Dimethiconol. Bevorzugt werden diese Dimethiconole in einer niedrigkonzentrierten Lösung in Cyclomethicone oder Dimethicone mit einer kinematischen Viskosität von 0,65 cSt bis maximal 10 cSt eingesetzt. Besonders bevorzugte Dimethiconole sind von Dow Corning unter den Handelsnamen Dow Corning 1401, Dow Corning 1403 und Dow Corning 1501 erhältlich; diese Produkte enthalten 10 bis 13 Gew.-% Dimethiconol in Cyclomethicone oder Dimethicone.

Erfindungsgemäß bevorzugte Zusammensetzungen sind dadurch gekennzeichnet, dass sie mindestens ein Silicongummi in einer Gesamtmenge von 0,01 - 0,5 Gew.-%, bevorzugt 0,05 - 0,2 Gew.-%, besonders bevorzugt 0,1 - 0,15 Gew.-%, jeweils bezogen auf das Gesamtgewicht der treibmittelfreien erfindungsgemäßen Zusammensetzung, enthalten.

Weitere bevorzugte erfindungsgemäße Zusammensetzungen sind dadurch gekennzeichnet, dass sie mindestens einen Duftstoff und/oder mindestens ein Parfümöl enthalten.

Als Duftstoffe oder Parfümöle können einzelne Riechstoffverbindungen, z.B. die synthetischen Produkte vom Typ der Ester, Ether, Aldehyde, Ketone, Alkohole und Kohlenwasserstoffe verwendet werden. Zu den phenolischen Riechstoffverbindungen zählt z. B. Carvacrol. Riechstoffverbindungen vom Typ der Ester sind z.B. Benzylacetat, Methylanthranilat, ortho-t-Butylcyclohexylacetat, p-tert.-Butylcyclohexylacetat, Diethylphthalat, Nonandiol-1,3-diacetat, iso-Nonylacetat, iso-Nonylformiat, Phenylethylphenylacetat, Phenoxyethylisobutyrat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat, Benzylsalicylat, Ethylsalicylat, iso-Amylsalicylat, Hexylsalicylat und 4-Nonanolid. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z.B. 6-Acetyl-1,1,3,4,4,6-hexamethyltetrahydronaphthalin, para-t-Amylcyclohexanon, 2-n-Heptylcyclopentanon, β-Methylnaphthylketon und die lonone α-Isomethylionon und Methylcedrylketon, zu den Alkoholen Zimtalkohol, Anethol, Citronellol, Dimyrcetol, Eugenol, Geraniol, Linalool und Terpineol, zu den Kohlenwasserstoffen gehören 1,3,4,6,7,8-Hexahydro-4,6,6,7,8,8-hexamethylcyclopenta-a-2-benzopyran, Hydroxymethylisopropylcyclopentan, 3-a-Methyldodecahydro-6,6,9a-trimethylnaphtho-2(2,1-b)furan, iso-Butylchinolin sowie die Terpene und Balsame.

Bevorzugt werden Mischungen verschiedener Riechstoffe verwendet, die gemeinsam eine ansprechende Duftnote erzeugen.

Geeignete Parfümöle können auch natürliche Riechstoffgemische enthalten, wie sie aus pflanzlichen oder tierischen Quellen zugänglich sind, z.B. Pinien-, Citrus-, Jasmin-, Rosen-, Lilien- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Melissenöl, Minzenöl, Zimtblätteröl, Lindenblütenöl, Wacholderbeerenöl, Vetiveröl, Olibanöl, Galbanumöl, Laudanumöl, Gewürznelkenöl, iso-Eugenol, Thymianöl, Bergamotteöl, Geraniumöl und Rosenöl.

Bevorzugte erfindungsgemäße Zusammensetzungen enthalten mindestens einen Duftstoff und/oder mindestens ein Parfümöl in einer Gesamtmenge von 0,1 - 10 Gew.-%, bevorzugt 0,5 - 8 Gew.-%, besonders bevorzugt 1 - 6 Gew.-%, außerordentlich bevorzugt 2 - 5 Gew.-%, jeweils bezogen auf das Gesamtgewicht der treibmittelfreien Zusammensetzung.

Weitere bevorzugte erfindungsgemäße Zusammensetzungen sind dadurch gekennzeichnet, dass sie mindestens einen verkapselten Duftstoff und/oder mindestens ein verkapseltes Parfümöl enthalten. Die Verkapselung kann dabei mit ähnlichen oder sogar denselben Bestandteilen erfolgen, wie sie vorstehend für die erfindungsgemäßen Wirkstoff-Kapseln beschrieben ist.

In einer weiteren bevorzugten Ausführungsform der Erfindung enthalten die erfindungsgemäßen Wirkstoff-Kapseln zusätzlich mindestens einen Duftstoff, der von den aromatischen Alkoholen der Struktur AA-1 verschieden ist. Besonders bevorzugt beträgt in solchen erfindungsgemäßen Wirkstoff-Kapseln das Gewichtsverhältnis von Alkohol/en der Struktur AA-1 zum mindestens einen, hiervon verschiedenen Duftstoff (1-500) : 1, bevorzugt (5-300) : 1, besonders bevorzugt (10-200) : 1, außerordentlich bevorzugt (50-150) : 1. Es kann aber auch bevorzugt sein, dass die erfindungsgemäßen Wirkstoff-Kapseln mehr Duftstoff ≠ AA-1 als Substanz(en) der Struktur AA-1 enthalten.

Weitere bevorzugte erfindungsgemäße Zusammensetzungen sind dadurch gekennzeichnet, dass sie mindestens einen als hautkühlend empfundenen Wirkstoff enthalten.

Erfindungsgemäß bevorzugte als hautkühlend empfundene Wirkstoffe sind beispielsweise Menthol, Isopulegol sowie Mentholderivate, z. B. Menthyllactat, Menthylpyrrolidoncarbonsäure, Menthylmethylether, Menthoxypropandiol, Menthonglycerinacetal (9-Methyl-6-(1-methylethyl)-1,4-dioxaspiro(4.5)decan-2-methanol), Monomenthylsuccinat und 2-Hydroxymethyl-3,5,5-trimethylcyclohexanol. Als hautkühlend empfundene Wirkstoffe bevorzugt sind Menthol, Isopulegol, Menthyllactat, Menthoxypropandiol und Menthylpyrrolidoncarbonsäure.

Bevorzugte erfindungsgemäße Zusammensetzungen enthalten mindestens einen als hautkühlend empfundenen Wirkstoff in einer Gesamtmenge von 0,1 - 3 Gew.-%, bevorzugt 0,2 - 2 Gew.-% und besonders bevorzugt 0,5 - 1 Gew.-%, jeweils bezogen auf das Gesamtgewicht der treibmittelfreien Zusammensetzung.

Weitere bevorzugte erfindungsgemäße Zusammensetzungen sind dadurch gekennzeichnet, dass sie mindestens einen als hautkühlend empfundenen Wirkstoff in verkapselter Form enthalten. Die Verkapselung kann dabei mit ähnlichen oder sogar denselben Bestandteilen erfolgen, wie sie vorstehend für die erfindungsgemäßen Wirkstoff-Kapseln beschrieben ist.

In einer weiteren bevorzugten Ausführungsform der Erfindung enthalten die erfindungsgemäßen Wirkstoff-Kapseln zusätzlich mindestens einen als hautkühlend empfundenen Wirkstoff. Besonders bevorzugt beträgt in solchen erfindungsgemäßen Wirkstoff-Kapseln das Gewichtsverhältnis von Alkohol/en der Struktur AA-1 zum mindestens einen als hautkühlend empfundenen Wirkstoff (1-500) : 1, bevorzugt (5-300) : 1, besonders bevorzugt (10-200) : 1, außerordentlich bevorzugt (50-150) : 1. Es kann aber auch bevorzugt sein, dass die erfindungsgemäßen Wirkstoff-Kapseln mehr als hautkühlend empfundenen Wirkstoff als Substanz(en) der Struktur AA-1 enthalten.

In einer weiteren besonders bevorzugten Ausführungsform sind die erfindungsgemäßen Zusammensetzungen frei von Ethanol. In einer weiteren besonders bevorzugten Ausführungsform sind die erfindungsgemäßen Zusammensetzungen frei von Isopropanol. In einer weiteren besonders bevorzugten Ausführungsform sind die erfindungsgemäßen Zusammensetzungen frei von Ethanol und Isopropanol.

Bevorzugte erfindungsgemäße Zusammensetzungen sind dadurch gekennzeichnet, dass sie zusätzlich zu dem mindestens einen deodorierend wirkenden aromatischen Alkohol AA-1 b) mindestens einen weiteren Deodorant-Wirkstoff enthalten. Bevorzugte derartige Deodorant-Wirkstoffe sind ausgewählt aus Geruchsabsorbern, desodorierend wirkenden Ionenaustauschern, keimhemmenden Substanzen, präbiotisch wirksamen Substanzen sowie Enzyminhibitoren und, besonders bevorzugt, Kombinationen der genannten Deodorant-Wirkstoffe.

Silicate dienen als Geruchsabsorber, die auch gleichzeitig die rheologischen Eigenschaften der erfindungsgemäßen Zusammensetzung vorteilhaft unterstützen. Zu den erfindungsgemäß besonders vorteilhaften Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Weitere vorteilhafte Geruchsabsorber sind beispielsweise Zeolithe, Zinkricinoleat, Cyclodextrine, bestimmte Metalloxide, wie z. B. Aluminiumoxid, sowie Chlorophyll.

Unter keimhemmenden oder antimikrobiellen Wirkstoffen werden erfindungsgemäß solche Wirkstoffe verstanden, die die Zahl der an der Geruchsbildung beteiligten Hautkeime reduzieren bzw. deren Wachstum hemmen. Zu diesen Keimen zählen unter anderem verschiedene Spezies aus der Gruppe der Staphylokokken, der Gruppe der Corynebakterien, Anaerokokken und Mikrokokken.

Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Weiterhin sind Phenol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva® SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) einsetzbar.

Weitere bevorzugte Deodorant-Wirkstoffe sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit sind hier die Wirkstoffe, die in den Offenlegungsschriften DE 10333245 und DE 10 2004 011 968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen.

Zu den Enzyminhibitoren gehören Stoffe, die die für die Schweißzersetzung verantwortlichen Enzyme, insbesondere die Arylsulfatase, β-Glucuronidase, Aminoacylase, Esterasen, Lipasen und/oder Lipoxigenase, hemmen, z. B. Trialkylcitronensäureester, insbesondere Triethylcitrat, oder Zinkglycinat.

Bevorzugte erfindungsgemäße Zusammensetzungen sind dadurch gekennzeichnet, dass der mindestens eine zusätzliche Deodorant-Wirkstoff ausgewählt ist aus Arylsulfatase-Inhibitoren, β-Glucuronidase-Inhibitoren, Aminoacylase-Inhibitoren, Esterase-Inhibitoren, Lipase-Inhibitoren und Lipoxigenase-Inhibitoren, α-Monoalkylglycerinethern mit einem verzweigten oder linearen gesättigten oder ungesättigten, gegebenenfalls hydroxylierten C₆ - C₂₂-Alkylrest, insbesondere α-(2-Ethylhexyl)glycerinether, präbiotisch wirksamen Komponenten, Trialkylcitronensäureestern, insbesondere Triethylcitrat, Wirkstoffen, die die Zahl der an der Geruchsbildung beteiligten Hautkeime aus der Gruppe der Staphylokokken, Corynebakterien, Anaerokokken und Mikrokokken reduzieren bzw. deren Wachstum hemmen, Zinkverbindungen, insbesondere Zinkphenolsulfonat und Zinkricinoleat, Organohalogenverbindungen, insbesondere Triclosan, Chlorhexidin, Chlorhexidingluconat und Benzalkoniumhalogeniden, quartären Ammoniumverbindungen, insbesondere Cetylpyridiniumchlorid, Geruchsabsorbern, insbesondere Silikaten und Zeolithen, Natriumbicarbonat, Lantibiotika, sowie Mischungen der vorgenannten Substanzen.

Weitere bevorzugte erfindungsgemäße Zusammensetzungen sind dadurch gekennzeichnet, dass der mindestens eine zusätzliche Deodorant-Wirkstoff in einer Gesamtmenge von 0,1 - 10 Gew.-%, bevorzugt 0,2 - 7 Gew.-%, besonders bevorzugt 0,3 - 5 Gew.-% und außerordentlich bevorzugt 0,4 - 1,0 Gew.-%, weiterhin bevorzugt 0,5, 0,6, 0,7, 0,8 und 0,9 Gew.-%, bezogen auf das Gesamtgewicht der Aktivsubstanz in der treibmittelfreien Gesamtzusammensetzung, enthalten ist. Antioxidative Stoffe können der oxidativen Zersetzung der Schweißkomponenten entgegenwirken und auf diese Weise die Geruchsentwicklung hemmen. Bevorzugte Antioxidantien sind Imidazol und Imidazolderivate (z. B. Urocaninsäure), Peptide, wie z. B. D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und weitere Thioverbindungen (z. B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl-, Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg bis µmol/kg), ferner Metallchelatoren (z. B. α-Hydroxyfettsäuren, EDTA, EGTA, Lactoferrin), Huminsäuren, Gallensäure, Gallenextrakte, Catechine, Bilirubin, Biliverdin und deren Derivate, ungesättigte Fettsäuren und deren Derivate (z. B. γ-Linolensäure, Linolsäure, Arachidonsäure, Ölsäure), Folsäure und deren Derivate, Hydrochinon und dessen Derivate (z. B. Arbutin), Ubichinon und Ubichinol sowie deren Derivate, Vitamin C und dessen Derivate (z. B. Ascorbylpalmitat, -stearat, -dipalmitat, -acetat, Mg-Ascorbylphosphate, Natrium- und Magnesiumascorbat, Dinatriumascorbylphosphat und -sulfat, Kaliumascorbyltocopherylphosphat, Chitosanascorbat), Isoascorbinsäure und deren Derivate, Tocopherole und deren Derivate (z. B. Tocopherylacetat, -linoleat, -oleat und -succinat, Tocophereth-5, Tocophereth-10, Tocophereth-12, Tocophereth-18, Tocophereth-50, Tocophersolan), Vitamin A und Derivate (z. B. Vitamin-A-Palmitat), das Coniferylbenzoat des Benzoeharzes, Rutin, Rutinsäure und deren Derivate, Dinatriumrutinyldisulfat, Zimtsäure und deren Derivate, Kojisäure, Chitosanglycolat und -salicylat, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Selen und Selen-Derivate (z. B. Selenmethionin), Stilbene und Stilben-Derivate (z. B. Stilbenoxid, trans-Stilbenoxid). Erfindungsgemäß können geeignete Derivate (Salze, Ester, Zucker, Nukleotide, Nukleoside, Peptide und Lipide) sowie Mischungen dieser genannten Wirkstoffe oder wasserfreie Pflanzenextrakte, die diese Antioxidantien enthalten, eingesetzt werden.

Als lipophile, öllösliche Antioxidantien aus dieser Gruppe sind Tocopherol und dessen Derivate, insbesondere Tocopherylacetat, und Carotinoide sowie Butylhydroxytoluol und Butylhydroxyanisol bevorzugt.

Die Gesamtmenge der Antioxidantien in bevorzugten erfindungsgemäßen Zusammensetzungen beträgt 0,001 - 10 Gew.-%, vorzugsweise 0,05 - 5 Gew.-% und insbesondere 0,1 - 2 Gew.-%, bezogen auf die gesamte treibmittelfreie Zusammensetzung.

Auch komplexbildende Stoffe können die deodorierende Wirkung unterstützen, indem sie die oxidativ katalytisch wirkenden Schwermetallionen (z. B. Eisen oder Kupfer) stabil komplexieren. Geeignete Komplexbildner sind ausgewählt aus den vorstehend genannten Komplexbildnern. Bevorzugte erfindungsgemäße Zusammensetzungen sind dadurch gekennzeichnet, dass sie mittels eines komprimierten Treibmittels aus der Verpackung ausgetragen werden. Erfindungsgemäß bevorzugte Treibmittel (Treibgase) sind ausgewählt aus Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, Dichlorfluormethan, Chlordifluormethan, Chlorfluormethan, 1,1,2,2-Tetrachlor-1-fluorethan, 1,1,1,2-Tetrachlor-2-fluorethan, 1,2,2-Trichlor-1,1-difluorethan, 1,1,2-Trichlor-1,2-difluorethan, 1,1,1-Trichlor-2,2-difluorethan, 2,2-Dichlor-1,1,1-trifluorethan, 1,2-Dichlor-1,1,2-trifluorethan, 2-Chlor-1,1,1,2-tetrafluorethan, 1-Chlor-1,1,2,2-tetrafluorethan, 1,1,2-Trichlor-2-fluorethan, 1,2-Dichlor-1,2-difluorethan, 1,2-Dichlor-1,1-difluorethan, 1-Chlor-1,2,2-trifluorethan, 2-Chlor-1,1,1-trifluorethan, 1-Chlor-1,1,2-trifluorethan, 1,2-Dichlor-1-fluorethan, 1,1-Dichlor-1-fluorethan, 2-Chlor-1,1-difluorethan, 1-Chlor-1,1-difluorethan, 1-Chlor-2-fluorethan, 1-Chlor-1-fluorethan, 2-Chlor-1,1-difluorethen, 1,1,1,3-Tetrafluorethan, Heptafluoro-n-propan, Perfluorethan, Monochlordifluormethan, 1,1-Difluorethan, und zwar sowohl einzeln als auch in Kombination.

Besonders bevorzugt sind Propan, n-Butan, iso-Butan sowie Mischungen dieser Treibmittel. Es hat sich auch gezeigt, dass der Einsatz von n-Butan als einzigem Treibmittel erfindungsgemäß besonders bevorzugt sein kann. Weiterhin bevorzugt sind auch 1,1-Difluorethan, Propan, n-Butan, iso-Butan sowie Mischungen dieser Treibmittel, insbesondere Mischungen aus 1,1-Difluorethan und n-Butan.

Das Gesamtgewicht an mindestens einem organischen Treibmittel beträgt bevorzugt 10 - 90 Gew.-%, besonders bevorzugt 40 - 85 Gew.-%, außerordentlich bevorzugt 50 - 80 Gew.-% und weiter bevorzugt 70, 72, 74, 76, 78, 82, 84 oder 86 Gew.-%, jeweils bezogen auf das Gesamtgewicht der Zubereitung, bestehend aus der erfindungsgemäßen Wirkstoffkapsel-Suspension und dem Treibmittel.

Auch Kohlendioxid, Luft, Sauerstoff, Stickstoff, Helium und Argon können im Sinne der vorliegenden Erfindung vorteilhaft als Treibmittel eingesetzt werden. Ihr Anteil wird im Vergleich zu den vorstehend genannten organischen Treibmitteln deutlich geringer gewählt und beträgt 1 - 10 Gew.-%, bevorzugt 2 - 9 Gew.-%, jeweils bezogen auf das Gesamtgewicht der Zubereitung, bestehend aus der erfindungsgemäßen Wirkstoffkapsel-Suspension und dem Treibmittel.

Als Behälter, insbesondere als Druckgasbehälter (für den Einsatz mit komprimiertem Treibmittel) sind Gefäße aus Metall (Aluminium, Weißblech, Zinn), geschütztem bzw. nicht-splitterndem Kunststoff oder aus Glas, das außen mit Kunststoff beschichtet ist, bevorzugt, bei deren Auswahl Druck- und Bruchfestigkeit, Korrosionsbeständigkeit, Befüllbarkeit wie auch ästhetische Gesichtspunkte, Handlichkeit, Bedruckbarkeit etc. eine Rolle spielen. Bevorzugt gewährleisten spezielle Innenschutzlacke die Korrosionsbeständigkeit.

Ein weiterer Gegenstand der vorliegenden Anmeldung ist die nicht-therapeutische Verwendung der erfindungsgemäßen Zusammensetzungen zur Reduzierung oder Maskierung von Körpergeruch.

Ein weiterer Gegenstand der vorliegenden Anmeldung ist ein nicht-therapeutisches Verfahren zur Reduzierung oder Maskierung von Körpergeruch, das dadurch gekennzeichnet ist, dass eine erfindungsgemäße Zusammensetzung in einer wirksamen Menge auf die Haut aufgetragen wird. Besonders bevorzugt verbleibt die erfindungsgemäße Zusammensetzung für eine Zeit von 1 Minute bis 24 Stunden, bevorzugt 2 bis 12 Stunden, auf der Haut.

Die nachfolgenden Beispiele sollen die Erfindung verdeutlichen, ohne sie hierauf zu beschränken. Alle Mengenangaben beziehen sich, soweit nicht anders vermerkt, auf Gew.-%.

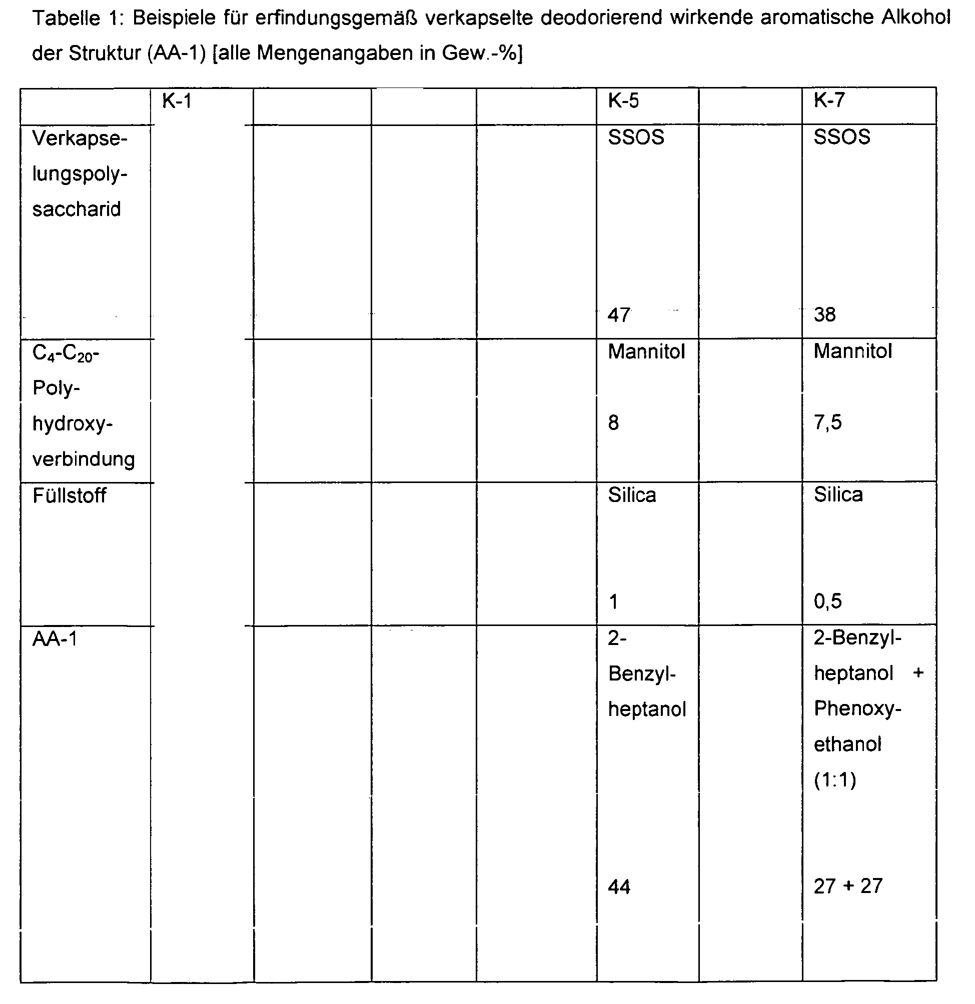

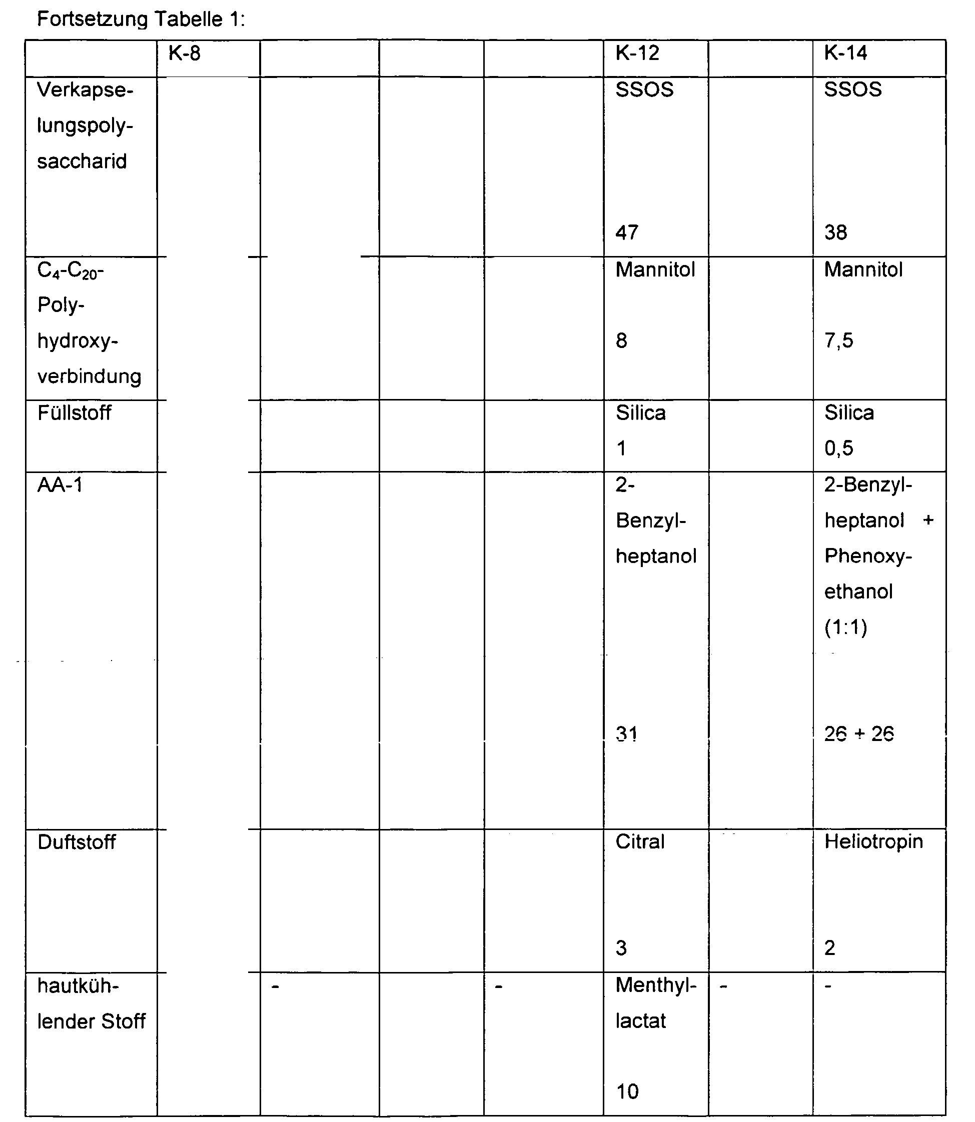

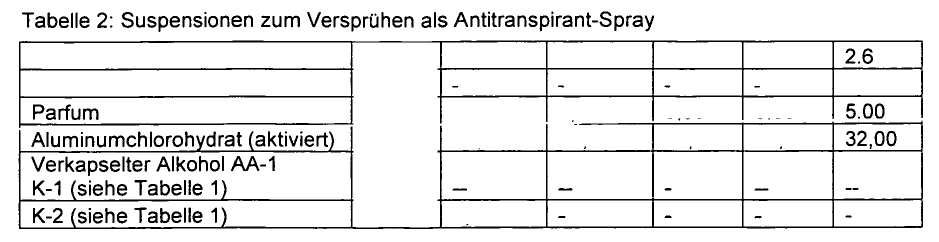

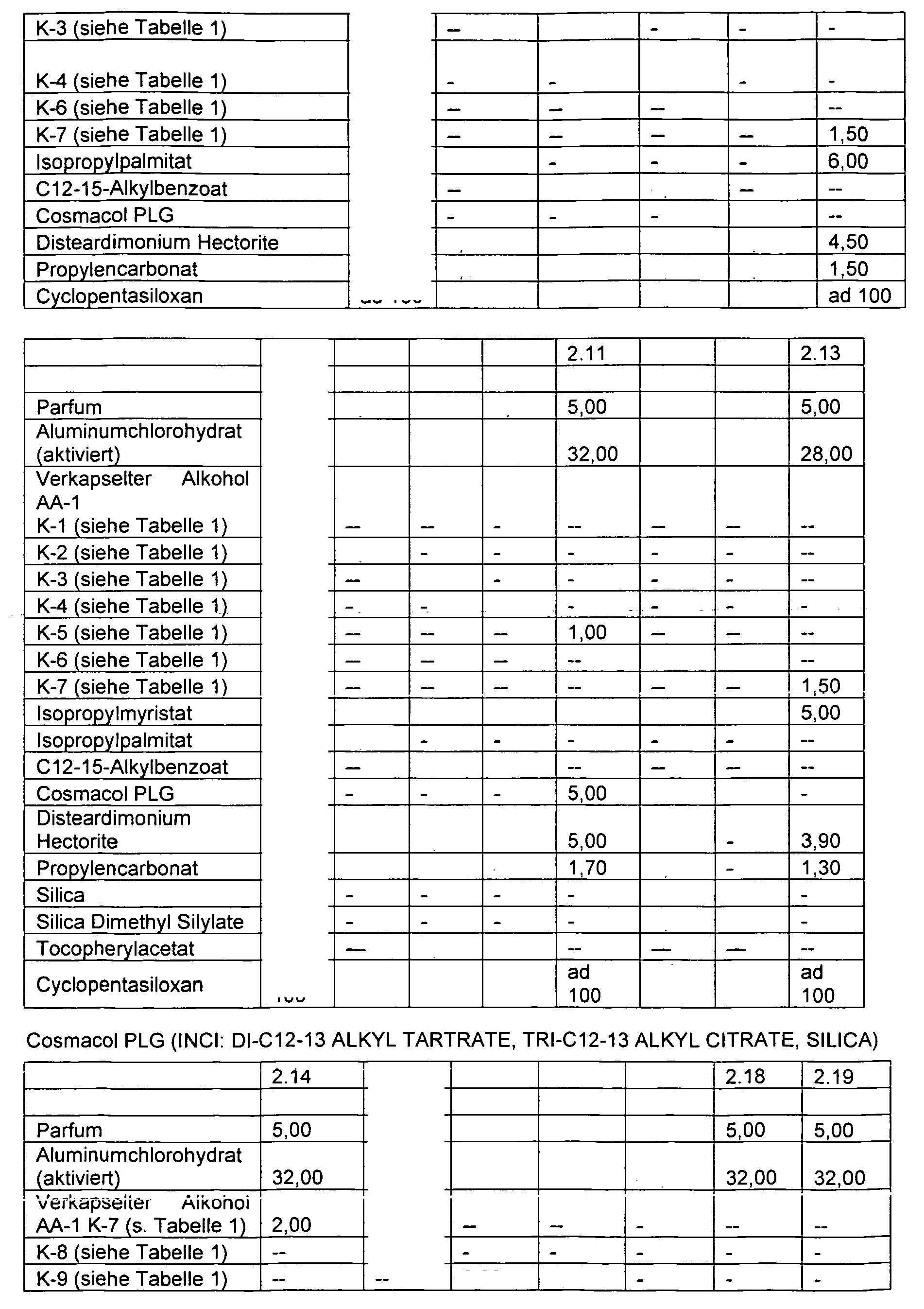

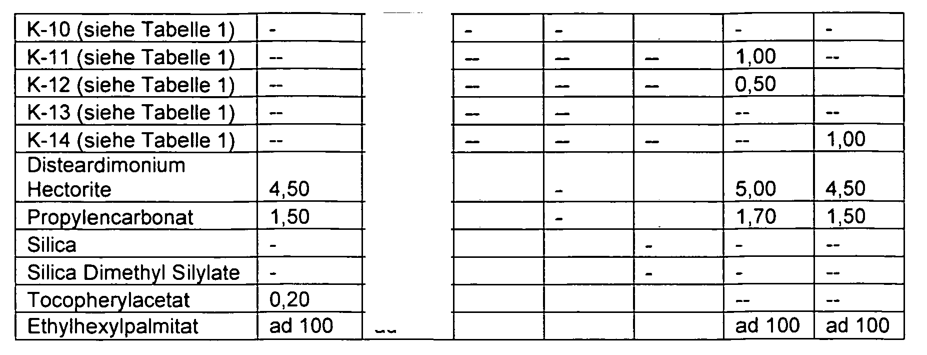

Die Zusammensetzungen 2.1 bis 2.19 wurden in Spraydosen aus innen lackiertem Aluminium abgefüllt und mit einer Isobutan/Butan/Propan-Treibmittel-Mischung im Gewichtsverhältnis Suspension : Treibmittel von 25 : 75, 22 : 78, 20 : 80, 18 : 82, 15 : 85 und 13 : 87 beaufschlagt; die erfindungsgemäßen Zusammensetzungen 2.12a und 2.16a wurden mit einer Butan/1,1-Difluorethan-Treibmittel-Mischung im Gewichtsverhältnis Suspension : Treibmittel von 45:55, 40:60 und 30:70 beaufschlagt.

**Tabelle 3: Suspensionen zum Versprühen als Deodorant-Spray**

| | | | | | |
|---|---|---|---|---|---|
| | 3.1 | | | | 3.5 |
| | | - | - | - | |
| Triethyl Citrate | 4,5 | | - | - | - |
| Parfum | 3,0 | | | | 3,0 |
| Tocopherylacetat | 0,2 | | | - | 0,2 |
| 2-Ethylhexylglycerin (Sensiva SC50) | 1,0 | | | | - |
| BHT | - | - | - | | - |
| Linalool | 0,15 | | | | 0,15 |
| Butylphenyl Methylpropional | 0,04 | | | | 0,04 |
| Phenoxyethanol | 1,5 | | | | - |
| K-7 (siehe Tabelle 1) | 2,00 | - | -- | - | -- |
| K-2 (siehe Tabelle 1) | -- | | - | - | - |
| K-3 (siehe Tabelle 1) | -- | -- | | - | - |
| K-4 (siehe Tabelle 1) | - | - | - | | - |
| K-5 (siehe Tabelle 1) | -- | -- | -- | -- | 1,00 |
| Triclosan | -- | -- | -- | -- | 0,3 |
| Ethanol (denat.) (wasserfrei) | ad 100 | | | | ad 100 |

Die erfindungsgemäßen Zusammensetzungen 3.1 bis und 3.5 wurden in Spraydosen aus innen lackiertem Aluminium abgefüllt und mit einer Isobutan/Butan/Propan-Treibmittel-Mischung im Gewichtsverhältnis Suspension : Treibmittel von 25 : 75, 22 : 78, 20 : 80, 18 : 82, 15 : 85 und 13: 87 beaufschlagt.

**Tabelle 4: Suspensionen zum Versprühen als Deodorant-Spray**

| | | | | | |
|---|---|---|---|---|---|
| | 4.1 | | | | 4.5 |
| | | - | - | - | |
| Triethyl Citrate | 1,5 | | - | - | - |
| Parfum | 1,0 | | | | 1,0 |
| Tocopherylacetat | 0,1 | | | - | 0,05 |
| 2-Ethylhexylglycerin (Sensiva SC 50) | 0,5 | | | | - |
| BHT | - | - | - | | - |
| Linalool | 0,05 | | | | - |
| Butylphenyl Methylpropional | 0,01 | | | | 0,01 |
| Phenoxyethanol | 0,5 | | | | - |
| Verkapselter Alkohol AA-1 K-7 (siehe Tabelle 1) | 0,5 | -- | -- | - | -- |
| K-2 (siehe Tabelle 1) | -- | | - | - | - |
| K-3 (siehe Tabelle 1) | -- | -- | | - | - |
| K-4 (siehe Tabelle 1) | - | - | - | | - |
| K-5 (siehe Tabelle 1) | -- | -- | -- | -- | 0,2 |
| Triclosan | -- | -- | -- | -- | 0,1 |
| Ethanol (denat.) (wasserfrei) | ad 100 | | | | ad 100 |

Die erfindungsgemäßen Zusammensetzungen 4.1 bis und 4.5 wurden ohne Treibmittel in einen Sprühspender abgefüllt.

Die erfindungsgemäßen Zusammensetzungen 2.1 bis 4.5 wurden von Probanden direkt nach der Körperreinigung auf ihre Achselhaut aufgesprüht.

Das Entfernen der erfindungsgemäßen Zusammensetzungen (beispielsweise durch Waschen) erfolgt im Rahmen der üblichen Hygieneroutine, beispielsweise 1, 2, 3, 4, 5, 6, 7, 8, 9, 10, 11, 12 oder 24 Stunden nach der Applikation.

## Patentansprüche

1. Wirkstoffkapseln mit deodorierender Wirkung, enthaltend
a) mindestens einen deodorierend wirkenden aromatischen Alkohol der Struktur (AA-1), 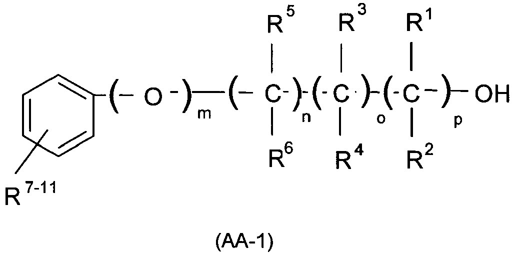
a)i) der verkapselt ist mit einer Mischung aus
1. mindestens einem Polysaccharid,
2. mindestens einer Polyhydroxyverbindung mit 4 bis 20 Kohlenstoffatomen, die unter Normalbedingungen fest ist,
3. mindestens einem teilchenförmigen Füllstoff, ausgewählt aus Kieselsäuren, Silikaten, insbesondere Schichtsilikaten, sowie Mischungen hiervon,
a)ii) wobei die Struktur (AA-1) folgendermaßen gekennzeichnet ist:
R¹ - R⁶ = unabhängig voneinander ein Wasserstoffatom, eine Alkylgruppe mit 1 bis 10 Kohlenstoffatomen, die linear oder verzweigt sein kann und die substituiert sein kann mit OH-Gruppen oder Alkoxy-Gruppen mit 1 bis 5 Kohlenstoffatomen, oder eine Alkenylgruppe mit 2 bis 10 Kohlenstoffatomen, die linear oder verzweigt sein kann und die substituiert sein kann mit OH-Gruppen oder Alkoxy-Gruppen mit 1 bis 5 Kohlenstoffatomen,
R⁷ - R¹¹ = unabhängig voneinander ein Wasserstoffatom, ein Halogenatom, insbesondere ein Chloratom, oder eine Alkylgruppe mit 1 bis 10 Kohlenstoffatomen, die linear oder verzweigt sein kann und die substituiert sein kann mit OH-Gruppen oder Alkoxy-Gruppen mit 1 bis 5 Kohlenstoffatomen, insbesondere mit einer Methoyxgruppe,
m = 0 oder 1 ist,
n, o, p = unabhängig voneinander ganze Zahlen von 0 bis 10 sein können, wobei mindestens einer der Werte n, o, p ≠ 0 ist,
**dadurch gekennzeichnet, dass** der deodorierend wirkende aromatische Alkohol a) der Struktur (AA-1) ausgewählt ist aus 2-Benzylheptan-1-ol, und der deodorierend wirkende aromatische Alkohol a) verkapselt ist mit einer Mischung aus Natriumstärkeoctenylsuccinat, Mannitol und Kieselsäure (INCI: Silica).

2. Produkt, bestehend aus
i) einem Sprühspender und
ii) einer in dem Sprühspender verpackten wasserfreien kosmetischen Zusammensetzung, die als versprühbare Suspension konfektioniert ist und
a) Wirkstoffkapseln gemäß Anspruch 1 und
b) einen wasserfreien, unter Normalbedingungen flüssigen Träger enthält.

3. Produkt gemäß Anspruch 2, **dadurch gekennzeichnet, dass** in der Zusammensetzung mindestens ein schweißhemmender Wirkstoff enthalten ist.

4. Produkt gemäß Anspruch 2 oder 3, **dadurch gekennzeichnet, dass** der wasserfreie Träger mindestens ein kosmetisches Öl umfasst.

5. Produkt gemäß Anspruch 2, 3 oder 4, **dadurch gekennzeichnet, dass** der wasserfreie Träger Ethanol und/oder 1,2-Propylenglycol umfasst.

6. Produkt gemäß Anspruch 2, 3, 4 oder 5, **dadurch gekennzeichnet, dass** der wasserfreie Träger mindestens ein Verdickungs- oder Suspendiermittel umfasst.

7. Nicht-therapeutische Verwendung eines Produkts gemäß einem der Ansprüche 2 - 6 zur Reduzierung oder Maskierung von Körpergeruch.

8. Nicht-therapeutisches Verfahren zur Reduzierung oder Maskierung von Körpergeruch, **dadurch gekennzeichnet, dass** eine wasserfreie kosmetische Zusammensetzung, die als versprühbare Suspension konfektioniert ist und
a) Wirkstoffkapseln gemäß Anspruch 1 und
b) einen wasserfreien, unter Normalbedingungen flüssigen Träger enthält,
mittels eines Sprühspenders in einer wirksamen Menge auf die Haut aufgetragen wird.

9. Verfahren nach Anspruch 8, **dadurch gekennzeichnet, dass** in der Zusammensetzung mindestens ein schweißhemmender Wirkstoff enthalten ist.

10. Verfahren nach Anspruch 8 oder 9, **dadurch gekennzeichnet, dass** der wasserfreie Träger mindestens ein kosmetisches Öl umfasst.

11. Verfahren nach Anspruch 8 bis 10, **dadurch gekennzeichnet, dass** der wasserfreie Träger Ethanol und/oder 1,2-Propylenglycol umfasst.

12. Verfahren nach Anspruch 8 bis 11, **dadurch gekennzeichnet, dass** der wasserfreie Träger mindestens ein Verdickungs- oder Suspendiermittel umfasst.

## Claims

1. Active ingredient capsules having a deodorant effect, containing
a) at least one deodorizing aromatic alcohol of the structure (AA-1), 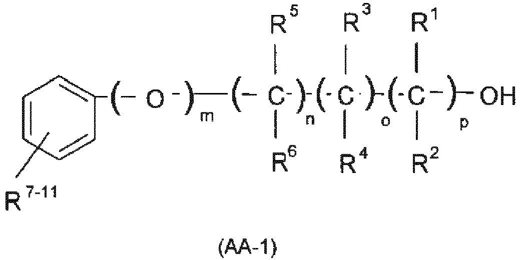
a)i) which is encapsulated by a mixture of
1. at least one polysaccharide,
2. at least one polyhydroxy compound which has from 4 to 20 carbon atoms and is solid under normal conditions,
3. at least one particulate filler, selected from silicic acids, silicates, in particular phyllosilicates, and mixtures thereof,
a)ii) the structure (AA-1) being characterized as follows:
R¹ - R⁶ are, independently of one another, a hydrogen atom, an alkyl group which has from 1 to 10 carbon atoms, can be linear or branched and can be substituted with OH groups or alkoxy groups having from 1 to 5 carbon atoms, or an alkenyl group which has from 2 to 10 carbon atoms, can be linear or branched and can be substituted with OH groups or alkoxy groups having from 1 to 5 carbon atoms,
R⁷ - R¹¹ are, independently of one another, a hydrogen atom, a halogen atom, in particular a chlorine atom, or an alkyl group which has from 1 to 10 carbon atoms, can be linear or branched and can be substituted with OH groups or alkoxy groups having from 1 to 5 carbon atoms, in particular with a methoxy group,
m = 0 or 1,
n, o, p can be, independently of one another, integers from 0 to 10, where at least one of the values n, o, p ≠ 0,
**characterized in that** the deodorizing aromatic alcohol a) of the structure (AA-1) is selected from and the deodorizing aromatic alcohol a) is encapsulated by a mixture of sodium starch octenylsuccinate, mannitol and silicic acid (INCI: silica).

2. A product consisting of
i) a spray dispenser and
ii) a water-free cosmetic composition which is packaged in the spray dispenser, is formulated as a suspension that can be sprayed and contains
a) active ingredient capsules according to claim 1 and
b) a water-free carrier which is liquid under normal conditions.

3. The product according to claim 2, **characterized in that** the composition contains at least one antiperspirant active ingredient.

4. The product according to one of claims 2 or 3, **characterized in that** the water-free carrier comprises at least one cosmetic oil.

5. The product according to one of claims 2, 3 or 4, **characterized in that** the water-free carrier comprises ethanol and/or 1,2-propylene glycol.

6. The product according to one of claims 2, 3, 4 or 5, **characterized in that** the water-free carrier comprises at least one thickening or suspending agent.

7. The non-therapeutic use of a product according to one of claims 2 to 6 for reducing or masking body odor.

8. A non-therapeutic method for reducing or masking body odor, **characterized in that** a water-free cosmetic composition which is formulated as a suspension that can be sprayed and contains
a) active ingredient capsules according to claim 1 and
b) a water-free carrier which is liquid under normal conditions,
is applied to the skin in an effective amount by means of a spray dispenser.

9. The method according to claim 8, **characterized in that** the composition contains at least one antiperspirant active ingredient.

10. The method according to one of claims 8 or 9, **characterized in that** the water-free carrier comprises at least one cosmetic oil.

11. The method according to one of claims 8 to 10, **characterized in that** the water-free carrier comprises ethanol and/or 1,2-propylene glycol.

12. The method according to one of claims 8 to 11, **characterized in that** the water-free carrier comprises at least one thickening or suspending agent.

## Revendications

1. Capsules de principe actif à effet désodorisant, contenant
a) au moins un alcool aromatique à effet désodorisant de structure (AA-1), 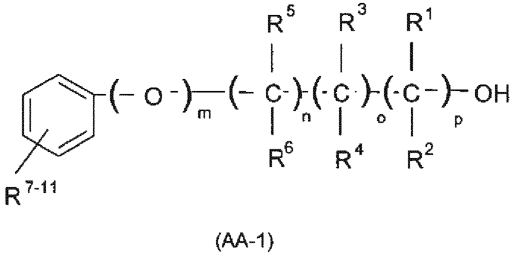
a)i) qui est encapsulé avec un mélange constitué
1. d'au moins un polysaccharide,
2. d'au moins un composé polyhydroxylé ayant de 4 à 20 atomes de carbone, qui est solide en conditions normales,
3. d'au moins une charge de forme particulaire, choisie parmi les acides siliciques, les silicates, en particulier les silicates lamellaires, et leurs mélanges,
a) ii), dans lesquelles la structure (AA-1) est **caractérisée** comme suit :
R¹ - R⁶ = indépendamment les uns des autres, un atome d'hydrogène, un groupe alkyle ayant de 1 à 10 atomes de carbone qui peut être linéaire ou ramifié et peut être substitué par des groupes OH ou des groupes alcoxy ayant de 1 à 5 atomes de carbone, ou un groupe alcényle ayant de 2 à 10 atomes de carbone, qui peut être linéaire ou ramifié et qui peut être substitué par des groupes OH ou des groupes alcoxy ayant de 1 à 5 atomes de carbone,
R⁷ - R¹¹ = indépendamment les uns des autres, un atome d'hydrogène, un atome d'halogène, notamment un atome de chlore, ou un groupe alkyle ayant de 1 à 10 atomes de carbone, qui peut être linéaire ou ramifié et qui peut être substitué par des groupes OH ou des groupes alcoxy ayant de 1 à 5 atomes de carbone, en particulier avec un groupe méthoxy,
m est égal à 0 ou 1,
n, o, p = indépendamment les uns des autres, peuvent être des nombres entiers allant de 0 à 10, où au moins l'une des valeurs n, o, p ≠ 0,
**caractérisées en ce que** l'alcool aromatique à effet désodorisant a) de structure (AA-1) est choisi parmi et l'alcool aromatique à effet désodorisant a) est encapsulé avec un mélange constitué d'octénylsuccinate acide d'amidon, sel de sodium, de mannitol, et d'acide silicique (INCI : silice).

2. Produit constitué
i) d'un pulvérisateur et
ii) d'une composition cosmétique anhydre conditionnée dans le pulvérisateur, qui est formulée en tant que suspension pulvérisable et
a) des capsules de principe actif selon la revendication 1 et
b) un support anhydre liquide en conditions normales.

3. Produit selon la revendication 2, **caractérisé en ce qu'**au moins un principe actif anti-transpirant est présent dans la composition.

4. Produit selon la revendication 2 ou 3, **caractérisé en ce que** le support anhydre comprend au moins une huile cosmétique.

5. Produit selon la revendication 2, 3 ou 4, **caractérisé en ce que** le support anhydre comprend de l'éthanol et/ou du 1,2-propylèneglycol.

6. Produit selon la revendication 2, 3, 4 ou 5, **caractérisé en ce que** le support anhydre comprend au moins un agent épaississant ou de mise en suspension.

7. Utilisation non-thérapeutique d'un produit selon l'une quelconque des revendications 2 à 6 pour réduire ou masquer les odeurs corporelles.

8. Procédé non-thérapeutique pour réduire ou masquer les odeurs corporelles, **caractérisé en ce qu'**une composition cosmétique anhydre, qui est formulée en tant que suspension pulvérisable et
a) contient des capsules de principe actif selon la revendication 1
b) et un support anhydre liquide en conditions normales,
est appliquée sur la peau en une quantité efficace au moyen d'un pulvérisateur.

9. Procédé selon la revendication 8, **caractérisé en ce qu'**au moins un principe actif anti-transpirant est présent dans la composition.

10. Procédé selon la revendication 8 ou 9, **caractérisé en ce que** le support anhydre comprend au moins une huile cosmétique.

11. Procédé selon les revendications 8 à 10, **caractérisé en ce que** le support anhydre comprend de l'éthanol et/ou du 1,2-propylèneglycol.

12. Procédé selon les revendications 8 à 11, **caractérisé en ce que** le support anhydre comprend au moins un agent épaississant ou de mise en suspension.
